# EUROPEAN PATENT APPLICATION

(11) **EP 4 556 046 A1**
(43) Date of publication of application: **21.05.2025**
(21) Application number: 23315425.1
(22) Date of filing: 16.11.2023
(51) Int. Cl.: A61M 25/01, A61M 25/09

(54) **A DELIVERY SYSTEM FOR A MEDICAL DEVICE, A GUIDEWIRE, A CATHETER DEVICE, A MEDICAL DEVICE, AND A METHOD OF TREATING A PATIENT**

(71) Applicant: Artedrone, 75013 Paris (FR)
(72) Inventor: The designation of the inventor has not yet been filed
(74) Representative: Hepp Wenger Ryffel AG

(57) **Abstract**

Delivery system (1) for a medical device (100). The medical device (100) has a controlling line (101) and a distal magnetic head (102). The controlling line (101) has a proximal portion (103) and a distal portion (104). At least the distal portion (104) is flexible and attached to the magnetic head (102). The delivery system (1) comprises at least a first actuator (2), a second actuator (3), a control unit (4), and a delivery channel (5) with a longitudinal axis (L). The delivery channel (5) is receiving the medical device (100) therein. The first actuator (2) is adapted for moving the proximal portion (103) of the controlling line (101) in at least two directions along the longitudinal axis (L). The second actuator (3) is exerting a force, along a distal direction of the longitudinal axis (L), on the distal magnetic head (102) and/or the distal portion (104).

## Description

The present invention relates to a delivery system for a medical device, and to a guidewire, a catheter device, a medical device, and a method of treating a patient, according to the preamble of the independent claims.

Delivery systems for delivering certain medical devices are known in the art.

New intravascular medical devices are emerging. Some emerging medical devices are flow-driven devices. New delivery systems to more easily manipulate these flow-driven devices are needed. Some of these flow-driven medical devices have a controlling line to control the motion of the medical device in the flow. The flow driven medical devices with a controlling line may be referred to called tethered medical devices.

For example, WO 2022/157189 discloses a system for moving an intravascular medical device in a vascular network. The system comprises a magnetic actuator, a controlling unit and a controlling line driver. The controlling line driver is adapted to hold and/or to release a controlling line part of the medical device at different speeds. The magnetic actuator is adapted to generate a magnetic field at a predetermined location in order to pull the medical device in a pre-determined direction.

WO 2021/009615 discloses a flow directed device adapted to be guided by a fluid flow, such as blood flow, within a tubular structure such as a blood vessel. The device comprises a proximal end, a distal end, and an elongated body in between. The distal end comprises a ferromagnetic area or structure capable of deflecting the distal end when subjected to an external magnetic field.

However, known systems have several disadvantages. In particular, it may be challenging to move a device which is attached to a controlling line within a channel of a delivery device due to the controlling line's low rigidity. Similarly, delivery devices known in the art may not be suitable for delivery of tethered medical devices.

Thus, the object of the present invention is to overcome the drawbacks of the prior art, in particular to provide a delivery system which allows a better control of the motion of tethered medical devices.

This and other objects are achieved by the medical implant and the methods according to the characterizing portion of the independent claims of the invention.

In the following, the invention is described with reference to tethered vascular medical device which may be steered in the vascular network through magnetic field (attraction or repulsion) controlled and generated by a magnetic guidance system around the patient and interacting with a magnetic component arranged with respect to a distal part of the device.

A controlling line, which may form the tether, may allow to interact physically with the medical device which which may be moving and operating in the vasculature.

Any controlling line mentioned herein may comprise or consist of a hollow tube, a wire, be formed by monofilaments or multifilaments, and/or may have several wires assembled, e.g., in parallel.

The length of the controlling line may be between 100 mm to 3000 mm, preferably between 250 mm and 2000 mm.

An outer diameter of the distal part of the controlling line is between 10 um and 900 µm, preferably between 60 µm and 700 um.

Tethered medical devices may have a distal part which is adapted to navigate in the vascular network. The distal part may have a magnetic element to steer and to be attracted by an external magnetic field the distal part. The distal part may be moved forward by a flow. A proximal part may be designed to remain outside of the patient and interact with a delivery device.

The magnetic guidance system may allow to control the different forces applied on the medical device during delivery and navigation. The guidance system may have a controller with a user interface used by the physician to control the medical device. Generally, the navigation system may comprise or be adapted to interact with an imaging system, a magnetic actuator used around the patient, a delivery system to which the controlling line is attached, a tracking system to monitor the patient, the magnetic actuator and imagery system movement.

The invention is directed to a delivery system which is suitable for delivering a medical device. The medical device is adapted for floating in or moving with a fluid in a patient's body, for example in blood. The medical device further has at least a controlling line and a distal magnetic head. The controlling line having a proximal portion and a distal portion and wherein at least the distal portion is flexible and attached to the magnetic head. The delivery system comprises at least a first actuator, a second actuator, a control unit, and a delivery channel having a longitudinal axis. The delivery channel is adapted for at least partially receiving the medical device therein. The first actuator is adapted for moving the proximal portion of the controlling line in at least two directions along the longitudinal axis, for example forward and backward. The second actuator is adapted for exerting a force, along a distal direction of the longitudinal axis, on the distal magnetic head and/or the distal portion of the controlling line. The control unit is adapted to control the first actuator depending on the second actuator.

In the following, delivery may in particular refer to moving the medical device through the delivery system up until the medical device reaches a patient's vasculature and floats in a blood stream, whereas navigation may refer to the navigation in a blood stream in particular with assistance of magnetic steering.

The proximal portion may be a rigid portion. It will be understood that a rigid portion of a controlling line is still highly flexible and is rigid in comparison to the distal portion of the flexible line. A rigid portion may be just rigid enough to allow pushing of the rigid portion.

The controlling line may comprise or consist of a hollow tube. The proximal section of the hollow tube may have an outer diameter between 0.3 mm and 4 mm, preferably between 0.9 mm and 2.5 mm. The inner diameter may be between 0.2 mm and 2 mm, preferably between 0.5 mm and 1.2 mm. The hollow tube may be made of metals, such as nickel-titanium alloys, or polymers, such as PTFE, Tygon, PEBAX, or a combination thereof. The hollow tube may be comprise several tubes arranged together. For example, the outer tube may be made of PEBAX while the inner tube is made of PTFE.

Additionally or alternatively, a portion of the hollow rigid structure may be more flexible than the remaining rigid structure. Preferably, the portion connects the remaining rigid structure with the proximal flexible part. This may be achieved by reducing the thickness of the tube, removing one inner tube, and/or by adding a flexible element made of an elastomer (silicone, polyurethane).

With a flow-driven tethered medical device, forces might not be transmitted from the proximal extremity of the medical device to the other sections of the medical device. Motion actuation might be needed on multiple sections of the medical sections at least on the proximal and distal sections of the tethered medical device. The blood flow may provide the main actuation of flow-driven medical devices. Accessory actuation methods may be necessary when the blood flow forces are not available or not strong enough to move the medical device. For that reason, two types of motion actuation may be present in the delivery system according to the invention, including a proximal actuation to move the proximal section of the medical device and a distal actuation to move the sections that cannot be activated by the proximal actuation. The proximal actuator may be referred as "first actuator". The distal actuator may be referred to as "second actuator". The two actuators might be synchronised to ensure a proper motion of the full medical device. In general, the first and/or the second actuator may be referred to as a controlling line actuator.

As a result, the delivery device according to the invention enable moving a medical device by interacting with the distal, more flexible portion of the controlling line, without putting constraints on the proximal part of the controlling line, in particular on the rigidity of the proximal part. For example, for certain applications, it may be preferable to have a proximal part which is more rigid than the distal part, for example when the controlling line is mechanically too flexible to allow pushing of the device. In such a case, it may also be preferably to use a first actuator adapted to push the proximal part of the controlling line. Such an adapted may have a limited suitability to move the flexible distal part, however. Because the second actuator may pull the controlling line, for example, using a push actuator is still possible and therefore enables the delivery device according to the invention to deliver a wide range of different tethered medical devices.

The delivery system may in particular perform, or assist in performing, an injection of the medical device into the patient's vascular network, control of the release of the controlling line (direction, orientation and speed) to control the displacement of the medical device, activation of functions of the medical device, such as removal of a protective coating, suction, solution injection, deployment of a guidewire, and/or distal sensors for connection and control.

The design of the delivery system may be adapted to the design of the medical device and its functions. Some features of the delivery system can be combined according to the design and functions of the medical device. In particular, the delivery device may be adapted to minimize and/or compensate for friction in the delivery system, e.g. friction of the controlling line on an inner wall of the delivery device.

The delivery channel may be in fluid connection with an introducer, preferably via a connector. The introducer may be an introducer catheter.

The introducer may have a user port which provides a connection point for the introducer and the connector. The user port may have at least two channels, wherein one channel is connected to the connector, and a second channel is a user channel. The user channel may allow the passage of a guidewire, the injection of a solution, e.g. link contrast or saline solution.

The user port may have a valve to give access to the introducer channel. For example, a guidewire may be used to move the introducer in the vascular network. In one configuration, the user channel has an hemostatic valve from its proximal side.

Furthermore, a inner surface of the introducer may be hydrophilic to minimize the friction in wet conditions. In particular, a hydrophilic layer such as PTFE may be used and/or a coating with hydrophilic molecules may be used. For example, a synthetic molecule such as PVA (PVA-6% w/w solution) or biological molecules such as albumin protein (an albumin-6% w/v solution) may be used for coating. The coating solutions may be sterilized before coating. After the coating, the introducer may be rinsed with a saline solution to remove unbound hydrophilic molecules. All the tubing in the delivery system may have the same hydrophilic property.

The introducer may have multiple channels to facilitate the operation of different tools in parallel. A first channel may be used for the delivery of the medical device. For example, an guidewire used to deliver the medical device at the extremity of the introducer may be introduced in a channel different from the first channel.

Once the delivery system is in operation, there may be no need for direct manipulation of the medical device and/or components of the delivery system. The medical device may be controlled remotely using a controller and a user interface.

The first and second actuators are adapted to control the motion of the controlling line. Different designs, which will be described herein, are suitable for this purpose. In general, forward motion (i.e. in a distal direction) may be achieved by releasing the controlling line, thus allowing the distal section of the medical device to advance in the vascular tree. Backward motion (i.e. in a proximal direction) may be obtained by retracting the proximal section of the controlling line bringing back the magnetic head of the medical device toward an introducer.

The first actuator may comprise or consist of at least one, preferably two, clamps. Additionally or alternatively, the first actuator may comprise rods and/or a spool mechanism.

If the controlling line is hollow and the first actuator comprises one or several clamps, the force exercised by the clamps to grab the controlling line may be adjusted to ensure that the hollow channel is not obstructed by the grabbing. In particular, the applied force could be adapted depending on the phase of the procedure, e.g. using a stronger force when moving the controlling line and a lower force when holding the controlling line. For example, a force between 0.001 N and 5 N, preferably between 0.1 N and 1 N, may be sufficient to compensate a drag force acting on the controlling line and thus to stop its motion.

The clamps may be made of metals such as stainless steel (e.g. AISI 316L) or polymers, e.g. polypropylene, or a combination thereof. The surface of the clamps may be coated with an elastomer, e.g. silicone, to improve grip and to avoid permanent deformation on the controlling line. The clamps may have a width between 2 mm and 50 mm, preferably between 5 mm and 20 mm. The length of the distal part of the clamp, e.g. a portion which is able to grip and hold the controlling line, may be between 1 mm and 30 mm, preferably between 7 mm and 15 mm. The proximal part of the clamp may be made of metals or polymers.

The delivery system may further comprise a fluid pump and/or a fluid reservoir. Preferably, the control unit is adapted for injecting and/or withdrawing fluid from the delivery channel, via a fluid inlet. Preferably, the control unit is thus adapted to provide a positive pressure or a negative pressure (relative to atmosphere), particularly preferably a pressure with a predefined value. For example, a positive pressure may be used to expel a fluid at a distal end of the medical device. It will be understood that a fluid may be a liquid or a gas.

Thus, fluid flow may be used to pull the medical device and act as the second actuator.

The pump may inject a solution at different stage of the navigation. The flow rate of the pump may be between 30 mL/min and 300 mL/min, preferably between 80 mL/min and 150 mL/min, during the delivery of the medical device through the introducer. During navigation, the flow rate may be between 20 mL/min to 100 mL/min. When the medical device is in a steady condition, a minimum flow may be kept between 1 mL/min and 20 mL/min to maintain tension on the controlling line and to avoid blood flow penetration and stagnation in the introducer or in the delivery system. Moreover, just before navigation, the flow rate may be increased up to 100 mL/min to 200 mL/min to put the line under tension and to remove direct contact of the controlling line with walls. The liquid injection may not only be used to keep the controlling line under tension but to minimize the dry friction of the controlling line with walls such as the walls of the tubing in the delivery system, of the catheter or the blood vessels.

Therefore, the second actuator may, preferably, be formed by the fluid pump and/or a fluid reservoir.

The second actuator may comprise or consist of a guidewire. The guidewire may have an element, at a distal end, which is adapted to interact with the medical device, in particular the magnetic head and/or the distal part of the controlling line. Said element may, preferably, comprise or consist of at least one of a magnetic element, preferably an electromagnetic element, a spool, pliers, a clamp, and/or a balloon-based mechanism.

Therefore, the delivery device may be adapted to grab a portion of the controlling line so as to pull it in a distal direction and/or to pull the device back in a proximal direction.

The magnetic element of the guidewire may generate a magnetic field along a substantially in the region of magnetic element. The magnetic element may have a length between 5 mm and 50 mm, preferentially between 10 mm to 20 mm. The magnetic element may comprise or consist of a permanent magnet. Additionally or alternatively, for example formed by a soft ferromagnetic alloy such as iron alloy. In this case, the magnetic head of the medical device may magnetize the soft ferromagnetic alloy. Hence, the two elements are magnetically attracted. The magnetic head of medical device may be detached from the guidewire by the drag force induced by the blood flow. The magnetic element may also be configured as an electro-magnet. The magnetic field may thus be stopped when a current is not applied. Once the magnetic field is removed, the magnetic head is no longer attracted to the magnetic guidewire. Another solution to detach the magnetic head from the electro-magnetic guidewire is to reverse the polarity of the electro-magnet by inversing the current powering the electro-magnet. For example, the magnetic field is generated by a magnetic coil powered by a current. To prevent corrosion, the magnetic element may be coated with a polymer (such as Parylene C), a ceramic (such as silica based, zirconium based, TiO2), or a metal (gold, silver). The magnetic guidewire may be at least partially radiopaque. The magnetic guidewire may be used to retrieve medical device back into the introducer from the vascular network. The magnetic guidewire may be hollow and may be moved along the controlling line, e.g. to follow the path of the controlling line in the vascular network and to unblock medical device if required.

The delivery system may further comprise an introducer. The introducer may be in fluid connection with the delivery channel. The second actuator may comprise or consists of at least one magnetic element, and/or a mechanical attachment element. The magnetic element and/or the mechanical attachment element may be arranged in the introducer, preferably at a distal region of the introducer. Particularly preferably, magnetic elements and/or mechanical attachment elements are arranged along the full length of the catheter device. The catheter may act as an introducer.

As a result, it is possible to actuate the distal part of the medical device at a distal-most position within the patient before the device enters the blood stream.

The invention is further directed to a delivery system for a medical device. The delivery system may be a delivery system as described herein above. The medical device may be adapted for floating in a fluid in a patient's body and has a controlling line and a distal magnetic head. The controlling line may have a proximal portion and a distal portion. At least the distal portion may be flexible and attached to the magnetic head. The delivery system comprises a first actuator, a delivery channel, and a control unit. The first actuator is adapted for releasing and/or drawing back the controlling line. The first actuator is at least partially arranged within the delivery channel and/or the housing, preferably wherein the housing forms part of the delivery channel. The delivery channel may also, additionally or alternatively, be attached to the housing such as to form an inner channel which is in fluid communication with an inner volume of the housing. The control unit is adapted to control the first actuator such as to release and/or draw back the controlling line, preferably via the inner channel.

The delivery channel may include a housing to host the first actuator or parts of the first actuator. The housing hosting some elements of the first actuator may be connected to the delivery channel.

For example, the first actuator may have a first element (e.g. a clamp) in the housing and a second element outside the housing. Alternatively, the entire first actuator is arranged inside the housing. A first element may be in the housing and a second element in the delivery channel (if not the housing is not formed as part of the delivery channel) as well. The second element may then also be adapted to move from the delivery channel into the housing and back.

The delivery device may thus allow actuation of a controlling line which is fully within the housing. The housing may be sealed fluid-tight and thus avoid contamination. In particular where the controlling line is flexible along its full length, it may be possible and thus particularly safe for patients to arrange the first actuator fully within the housing.

The elements of the delivery system in contact with the medical device may be sterile. A subset of the delivery system may be delivered as a standalone sterile disposable or sterilizable component.

The housing may be a spool chamber and the controlling line actuator may be a spool.

The controlling line actuator may be filled with a biocompatible fluid which is suitable to be directly connected to the blood volume through the delivery channel and the introducer. This may avoid any transition or valve that may increase friction on the controlling line and limit or prevent the movement of the medical device/controlling line.

A flow sensor may be arranged within or connected with respect to the housing. The flow sensor may measure a velocity between 0.01 cm/s and 120 cm/s, preferably between 0.1 cm/s and 60 cm/s. The flow sensor may be, for example, connected at an exit port of the housing toward a connector or introducer.

The flow velocity may be measured by ultrasound or heat dissipation. These measurement techniques allow for sensors to be placed remotely and thus avoid obstructing the flow by the placement of sensors in the flow. Alternatively, the flow sensor may be arranged in parallel with respect to the connector or delivery channel. The flow sensor may measure the inflow and the backflow. The flow sensor may be used to detect an at least partial occlusion of the delivery system, e.g. of the introducer.

A pressure sensor may be arranged within or connected with respect to the spool chamber. The pressure sensor may be used to monitor the pressure in the chamber. For example, this monitoring may allow to confirm that the spool chamber is appropriately filled and/or that a component such as the introducer is not occluded or not opened/leaking.

The delivery channel and/or the housing may be in fluid communication with a purge valve.

A purge valve may avoid the presence of air bubbles. The purge valve may in particular be arranged on a top portion of the housing. To remove the air bubbles, for example, the purge valve may be opened and solution may be injected while verifying whether drops come out of the purge valve.

The housing may be filled with a saline solution. The saline solution may comprise heparin or a contrast agent. The saline solution may be adapted to be viscous and have a viscosity is between 1.1 mPa.s and 4 mPa·s, preferably between 1.1 mPa·s and 2 mPa·s).

The delivery system may further comprise a fluid pump and/or a fluid reservoir. Particularly preferably, the control unit may be adapted for injecting and/or withdrawing fluid from the delivery channel and/or the housing, via a fluid inlet. Preferably, the control unit is thus adapted to provide a positive pressure or a negative pressure (relative to atmosphere), particularly preferably a pressure with a predefined value. For example, a positive pressure may be used to expel a fluid at a distal end of the medical device. It will be understood that a fluid may be a liquid or a gas

Thus, fluid flow may be used to pull the medical device and act a second actuator. However, the fluid flow may also be used, e.g., to compensate for fluid exiting the delivery device into the patient's blood stream.

The controlling line actuators and the fluid pump may be controlled with the control unit, and/or each with a separate dedicated controller having a user interface. The control unit may allow to activate the controlling line actuator and the fluid pump simultaneously or sequentially. The control unit may record the parameters applied for the displacement of the medical device. The fluid pump may be used without the controlling line actuator to remove a slack of the controlling line generated during the navigation. The slack may in particular be a portion of the controlling line which is not under tension.

Further to avoid slack, in particular in the delivery channel, alignment of an exit channel with the motion system of the controlling line may be implemented. The exit channel may be in the center of the motion system and at the same level. Additionally or alternatively, a tapered exit channel may be used, in particular to avoid slack in the motion system by forcing slack to be present in the tapered area, if at all. Additionally or alternatively, a radius of a spool (which may be part of the controlling line actuator) may be at least five times the radius of the controlling line and/or the length of the spool may be between 10 and 80 times the diameter of the controlling line, preferably between 20 to 40 times.

To avoid slack in the introducer, a magnetic sensor in the introducer may be used. If slack is detected, the distal part of the medical device (in particular the magnetic head) may be moved back into the introducer and the accuracy of the displacement is checked between two sensors. Additionally or alternatively, the control unit may trigger a flush, i.e. an injection of a solution at a high flow rate, for example between 100 mL/min and 300 mL/min during a predetermined time, preferably between 2 s and 60 s.

A magnetic sensor to detect slack may include two sensors inside the lumen of the catheter. The magnetic head of the medical device may be placed at a position of the first sensor. The delivery system may then release a portion of controlling line corresponding to the distance between the first and the second sensor. If the magnetic head reaches the center of the second sensor, the magnetic field disturbance will match the theoretical model. If not, the disturbance of the magnetic field will be different, thus indicating that the release length is not accurate.

An automated flush may also be used to avoid slack in the vascular network. Additionally or alternatively, the control unit may operate the delivery system to displace the magnetic head over a determined length, for example over 5 mm to 30 mm and compare, from interventional images, whether the navigated distance corresponds to the calculated distance. If the difference is above a threshold value, e.g. 5%, the control unit may identify that slack has been created and then an automated flush may be triggered with the release of the medical device on a specific length, preferably 10 mm to 60 mm.

Thereby, inadequate deployment of the medical device may be avoided, e.g. due to wrong position of the medical device with respect to the targeted position. Also, knot formation in the controlling line may be avoided.

The housing may be continuously filled with the saline solution by using the pump and/or from the reservoir filled with the solution, e.g. a bottle or a bag. The flow rate in the housing may be regulated. The flow rate of the pump may be tuned with the controller of the pump. Alternatively, e.g. if only a fluid reservoir is present, an adjustable valve may be used. The movement of the medical device may be synchronized with the flow provided by the pump. The control unit, which may be adapted to control the motor and the pump, may be placed a cart away from the operating table.

The first actuator may comprise or consist of at least a first holder. Preferably, the first holder is arranged external of the delivery channel.

For example, a clamp, a tweezer, or another mechanical attachment element may be used to hold the controlling line. The holder may be stationary or movable.

Additionally or alternatively, the first actuator may comprise or consist of at least a second holder. Preferably, the second holder is arranged inside the delivery channel or/and in the housing.

The control unit may be adapted for controlling the operation of the first and/or second holder. Preferably, the control unit is adapted for controlling the first and/or second actuator dependent on the movement of the controlling line. The control unit may also, additionally or alternatively, be adapted for controlling the first and/or second actuator dependent on the respective other of the first and/or second actuator.

For example, the control unit may be adapted to detect if the controlling line is put under tension and thus release, if no tension is desired, for example. In other cases, tension may be an indication that the medical device is acting on a body element (e.g. pulling on a thrombus) and may adapt the pulling force accordingly.

Similarly, if the controlling line becomes loose, the control unit may slow down the release and/or draw the line back.

The delivery system may comprise an introducer. The introducer may be formed by a catheter. The introducer may be connected or connectable to the delivery channel by a connector. The connector may have an inlet.

The first actuator may comprise or consist of a spool.

The spool may be arranged within the housing.

The delivery system may further comprise a motor for operating the first actuator. The motor may be arranged outside the delivery channel and/or the housing. Additionally or alternatively, the motor may be in operable connection with the first actuator via a contactless gear and/or a rotary seal.

Therefore, even when the housing is fluid-tight and sealed, the motor may be accessed without opening the housing and/or the delivery channel, e.g. for maintenance.

The delivery channel and/or housing may, preferably, have a hemostatic valve adapted for receiving the controlling line and allowing sliding of the controlling line in a sealed manner.

In some circumstances, it may be desirable and/or necessary to introduce the controlling line into the housing during delivery. The hemostatic valve allows inserting the controlling line, in particular a rigid part of the control line, without inserting unwanted substances into the delivery channel and/or housing which may jeopardize the treatment.

The first actuator may further comprise at least one rod arranged within the delivery channel and/or the housing and adapted for tensioning and/or releasing the controlling line.

The rod, or plurality of rods, may in particular by used to release or retract the controlling line. To this end, the controlling line may first be loaded into the delivery channel or housing from the distal side, i.e. via the delivery channel.

The delivery system may further comprising a medical device. The medical device may be adapted for floating in a fluid in a patient's body, for example blood, and has a controlling line and a distal magnetic head. The controlling line has a proximal portion and a distal portion. At least the distal portion is flexible and attached to the magnetic head. The proximal portion of the controlling line is in operable connection with the first actuator. The proximal portion may in particular have a rigidity higher than the rigidity of the distal part and said rigidity being suitable for pushing the proximal part in a distal direction.

The controlling line, preferably the distal portion of the controlling line, of said medical device may be in operable connection with the second actuator.

At least a portion of an inner surface of the delivery channel and/or the housing is coated with an anti-friction coating in any delivery system disclosed herein.

The control unit is adapted for operating the first actuator and/or the fluid pump in dependence of a magnetic force acting on the medical device by a magnet adapted for steering the medical device in a patient's body.

For example, if an external magnet is operated to move the medical device at certain speed, the control unit may control the first actuator and/or fluid pump to match said speed when moving the controlling line. Similarly, if the external magnet is operated to position the magnetic head at a certain position, the control unit may control the first actuator and/or fluid pump so that the control line allows movement to said position.

The invention is further directed to a guidewire for arrangement or introduction in the introducer of a delivery device. The delivery device may be any delivery device as disclosed herein. The guidewire has a first end adapted for interacting with the medical device such as to pull or push the medical device through the introducer. The first end may comprise one of a magnet and a clamp.

The invention is further directed to a catheter device for delivering a medical device into a patient's body. The medical device has a magnetic head portion and a controlling line and is adapted for floating in a fluid in a patient's body.,

The catheter device may comprise a plurality of selectively activatable magnetic elements arranged along a longitudinal axis of the catheter device.

Therefore, the position and/or speed of the medical device may be controlled within the catheter device. For example, the medical device may be pulled through the catheter device by interaction of the magnetic elements with the magnetic head of the medical device.

The catheter device may further comprise a holder adapted to hold a controlling line. The holder may be arranged at a distal end of the catheter device.

The delivery channel may be connected to a catheter device. The delivery device which has the delivery channel may be any delivery device as disclosed herein. Similarly, the catheter device may be any catheter device disclosed herein. The catheter device may be an introducer as described above. An inner channel of the catheter device may form, at least partially, an extension of the delivery channel.

A continuous channel may be formed,by the inner channel and the delivery channel.

The invention is further directed to a medical device for introduction into a patient's body. The medical device comprises a magnetic head portion and a controlling line. A plurality of magnetic elements are arranged on or in the controlling line along a longitudinal axis. Particularly preferably, the magnetic elements do not substantially affect the mechanical properties of the controlling line.

Magnetic elements provide a particularly simple way for an actuator, for example magnetic elements within a distal part of a catheter device or introducer, to interact with the controlling line. Furthermore, it may be possible to exert a force selectively on different parts of the controlling line, e.g. by a magnet on a guidewire, magnetic elements within a catheter, or similar.

To pull the controlling line in the delivery system, an electro-magnet (e.g. of a guidewire) may be switched off and positioned close to one magnetic element of the controlling line, for example the closest to the distal part of the delivery channel. Then the electro-magnet may be switched on and may attract the magnetic element of the controlling line. Subsequently, the delivery system control unit may release the controlling line at a velocity between 0.5 cm/s and 50 cm/s, preferably between 2 cm/s and 10 cm/s, and simultaneously push forward the electro-magnetic guidewire to pull the distal section of the controlling line. The distal section of the controlling line and the medical device may then move with the flow (having a rate of 10 mL/min to 300 mL/min). Once the electro-magnetic part of the guidewire reaches the extremity of the delivery channel, the delivery system control unit may stop the release of the controlling line and the motion of the electro-magnetic guidewire. The controlling line stops moving forward. Once the electro-magnetic element has reached the extremity of the delivery channel and/or introducer, the electro-magnet may be switched off, the guidewire may be pulled back to the next magnetic element of the controlling line and a new pulling cycle may be started, as described above.

The delivery system as described above may also comprise a medical device as described above.

The invention is further directed to a method of treating a patient. The method comprises the step of introducing a medical device in a patient's body. The medical_device is adapted for floating in or moving with a fluid in a patient's body and has a controlling line and a distal magnetic head. Preferably, the medical device is a medical device as described herein above. The controlling line has a proximal portion and a distal portion. At least the distal portion is flexible and attached to the magnetic head. Particularly preferably, the method is performed using any one of the delivery systems as described herein above. The medical device motion may be synchronized with the heart cycle (diastole, systole). For example, the navigation and/or advancement may be started during a systole or diastole phase.

In general, to retract the controlling line, the delivery system may pull back the controlling line, in particular with the assistance of an electro-magnetic guidewire. The control unit may be operated to pull back the guidewire simultaneously.

Preferably, the movement of the guidewire and the controlling line are synchronized.

When there is no synchronization, the electro-magnet of the guidewire may be switched off and may be initially retracted by a shorter distance and remain positioned close to the extremity of the delivery channel and/or the introducer for a future release the controlling line.

The controlling line and the electro-magnetic guidewire may be placed and delivered in the same lumen of a delivery catheter/delivery channel/introducer or in different lumens of the delivery catheter/delivery channel/introducer each.

The control of the positioning of the electro-magnetic head of guidewire and/or of the magnetic elements of the controlling line may be done under fluoroscopy, using a localization sensor, and/or motion models.

The guidewire according to the invention may in particular be equipped with multiple electro-magnets that are adapted grab multiple magnetic elements of the controlling line.

It would also be conceivable to arrange a permanent magnet on the guide wire and/or use electro-magnetic elements distributed along the controlling line.

The magnetic elements of the controlling line may be distributed on the controlling line far enough one from the other to avoid attachment of the magnetic element among themselves. Preferably, to avoid such attraction, the handles are made of soft ferromagnetic materials.

A liquid which is delivered in the introducer may, in some embodiments, not be delivered by the pump of the delivery system but instead a with saline bag known in the art. The perfusion may, for example, be connected to the introducer through the user port.

The delivery system may have channels connected to the delivery system placed and configured to not be on the top of the delivery system. For example, the channels may be arranged at the center of the device. Additionally or alternatively, a purging valve may be arranged on a top portion of the delivery system e.g. on a housing or on the delivery channel. The purge valve may trap air bubbles and allow their release by opening the purge valve, as the air bubbles may be pushed out by injection a solution in the delivery system.

The delivery system may have one or several sensors such as a pressure sensor and/or a flow sensor. A pressure sensor may be placed in the delivery system where the solution is passing through, e.g. in the motorized spool chamber. The pressure sensor may be connected by any means known in the art, such as luer-lock connection or screwing. The pressure sensor may allow to detect an irregular increase of the pressure due to, e.g., an occlusion of the introducer and/or the non-opening of a valve. A pressure sensor may be placed before, i.e. upstream, of the introducer. The pressure sensor may be used to monitor the proximal pressure.

A flow sensor, in particular an ultrasound sensor, may be placed before the introducer. The flow sensor may allow the monitoring of the blood flow velocity in the introducer. The sensor may monitor backward and forward flow.

The delivery system, in particular the control unit, may be adapted to operate other device or elements, e.g. elements and devices which may interact with the medical device. For example, the control unit may operate sensors and/or control power supply for the medical device.

In the following, the invention is described in detail with reference to the following figures, showing:
- Fig. 1a-1c:: different examples of tethered medical devices.
- Fig. 2:: schematically a components of a delivery system.
- Fig. 3:: schematically a delivery system being operated.
- Fig. 4:: an embodiment of a delivery system.
- Fig. 5:: a detailed view of panel A of Fig. 4.
- Fig. 6:: a delivery system with clamps.
- Fig. 7:: operation of the delivery system of Fig. 6 to release the controlling line.
- Fig. 8:: operation of the delivery system of Fig. 6 to withdraw the controlling line.
- Fig. 9:: a re-arming process of the delivery system of Fig. 6.
- Fig. 10:: a delivery system having rods associated with the first actuator.
- Fig. 11:: operation of the delivery system of Fig. 10.
- Fig. 12:: an embodiment of a delivery channel with motion rods.
- Fig. 13:: the operation of the system of Fig. 12 to release the controlling line.
- Fig. 14:: the operation of the system of Fig. 12 to pull back the controlling line.
- Figs. 15a-15b:: a medical device having a controlling line with a distal and a proximal part.
- Fig. 16a-16b:: further embodiments of a delivery system.
- Figs. 17a-17c:: a further embodiment of a delivery system.
- Fig. 18:: a further embodiment of a delivery system.
- Figs. 19a-19b:: a delivery system with a microcatheter.
- Figs. 20a-20b:: a delivery system with a guidewire.
- Fig. 21:: a magnetic guidewire.
- Fig. 22:: an electromagnetic guidewire.
- Fig. 23:: a delivery system, partially shown, having a guidewire actuator.
- Fig. 24:: a system for treating a patient.
- Figs. 25a-25d:: a magnetic catheter device.
- Fig. 26:: a second embodiment of a magnetic catheter device.
- Fig. 27:: schematically the segments of a controlling line.
- Fig. 28:: a delivery system with a medical device having a controlling line with magnetic elements to be pulled by a magnetic guidewire.
- Fig. 29:: action of a magnetic guidewire to pull the medical device.
- Fig. 30:: a delivery system with a guide wire with mechanical attachment element.
- Figs. 31a-31d:: different embodiments of guidewires.
- Fig. 32:: a delivery system with a distal attachment element.
- Fig. 33:: the operation of the delivery system of Fig. 32 to actuate the controlling line.
- Fig. 34:: a delivery system for fluid circulation in the introducer.
- Figs. 35a-35b:: schematically two magneto-fluidic medical devices.
- Fig. 36:: schematically a first medical device being moved in a fluid.
- Fig. 37:: schematically a second medical device being moved in a fluid.
- Fig. 38:: schematically a medical device being pulled back.
- Figs. 39a-39b:: schematically different embodiments of a delivery system.
- Figs. 40a-40c:: schematically different arrangements of a proximal actuator.
- Fig. 41a-41c:: schematically two different proximal actuation mechanisms.

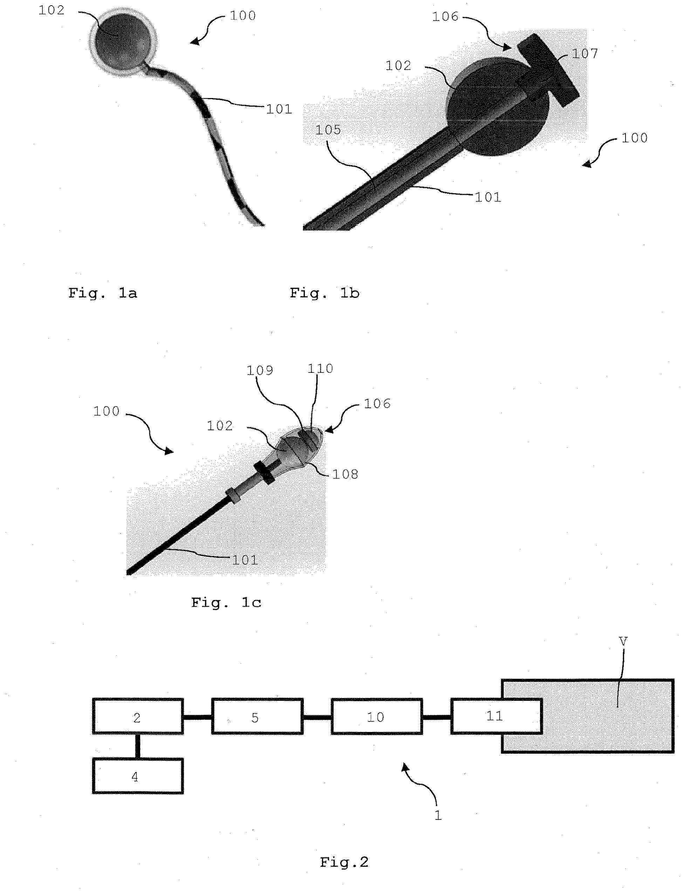

Fig. 1a shows a medical device 100 having a magnetic head portion 102 which is attached to a controlling line 101. The shown medical device 100 may be used as guide device which is adapted to navigate tortuous vascular networks. The controlling line 101 is used to control or influence the displacement of other medical devices (not shown) such as microcatheters or a guidewire.

Fig. 1b shows a medical device 100 comprising a suction cup 106 configured to attach to tissue, such as a thrombus (not'shown), via a suction action. The medical device 100 comprises a magnetic head 102 and a controlling line 101, both of which comprise a hollow portion forming an inner channel 105 which extends to, and is in fluid communication with, a suction hole 107 on the suction cup 106. Thus, here, mechanical attachment is performed by suction. The suction cup 106 may also have a flat shape, a curved shape, and/or comprise peripheral protrusions to provide sufficient fit with a shape of the thrombus. Here, a diameter of the opening 107 is 600 µm. The thrombus (not shown) can be pulled into suction cup 106 and be held by means of the suction. A suction mechanism as shown here allows to attach and reattach a thrombus repeatedly, for example until secure attachment is provided. Here, inner channel 105 has an inner diameter of 50 um. The controlling line 101 shown here is formed by a micro hollow tube and may, in addition to suction, be used to inject a solution, to remove and element by aspiration, and/or to deliver a component such as a metallic wire.

Fig. 1c shows a medical device 100 which comprises a magnetic part 102 with a tool support 109 which is configured with a chemical attachment element 110, here in the form of the drop of cyanoacrylate adhesive. In addition, the medical device 100 shown here comprises a protection cap 108 which consists of a capsule of thin foil of PVDF. Additionally or alternatively, PMMA and/or PA66 may be used. The protection cap 108 is retractable from the microrobot magnetic head 102 and adhesive drop 110 by being pulled in a proximal direction. The retracta-tion can cause tearing of the protective cap 108 leading to its opening. The protective cap 108 may avoid exposure of the adhesive 110 with blood as well as accidental attachment to a vessel wall. The shown device is particularly suited for the treatment of strokes by retrieving a thrombus blocking a vascular network. It will be understood that alternative attachment mechanisms are conceivable. For example, a retrieving tool setup on the distal part of the medical device may have a retrieving tool formed by a mechanical, chemical or biological anchoring mechanism for the thrombus.

The devices of Figs. 1a-1c are suitable to be delivered and/or guided using a delivery system as disclosed herein.

Fig. 2 shows schematically and in exemplary fashion components which may constitute a delivery system 1. An introducer 11 has a distal part which is placed in a vascular network V. A connector 10 may be used to connect the introducer 11 to the delivery channel 5. Via the connector 10, other interventional radiology elements such as a saline solution, a guidewire, and/or a contrast solution may also be introduced. The delivery channel 5 may be used to connect a controlling line actuator 2 to the introducer 11. The controlling line actuator 2 controls the motion and speed of the controlling line (not shown). A control unit 4 may operate the controlling line actuator 2.

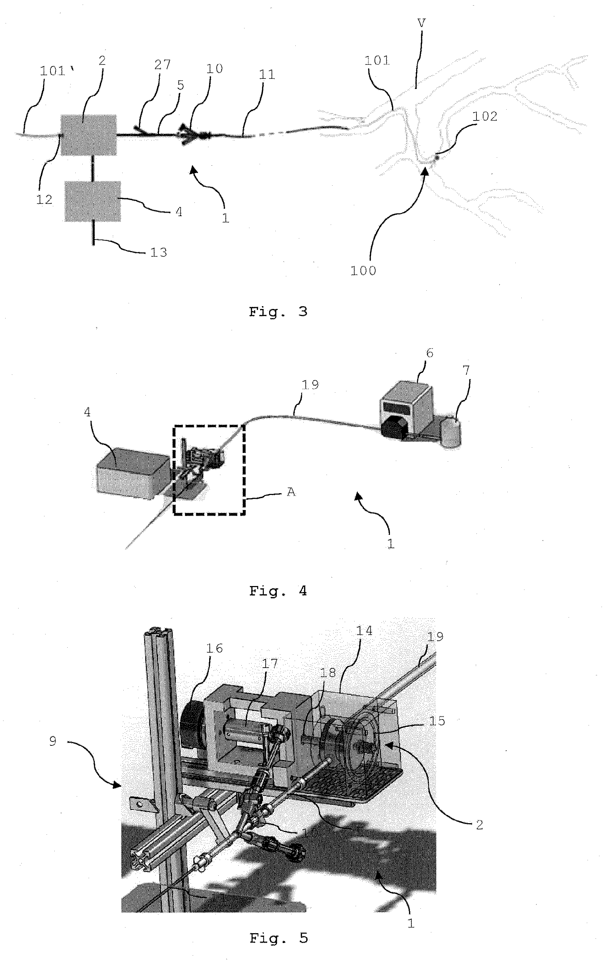

Fig. 3 shows, in a more detailed fashion, the operating principle of the delivery system 1. The introducer 11 is inserted in the patient vasculature V up to a location where a medical device 100 is able to navigate autonomously. The delivery system 1 is generally similar to the delivery system of Fig. 2. The connector 10 connects introducer 11 to delivery channel 5. As illustrated, the connector 10 has two additional ports, for example for introduction of a guidewire to set or move the introducer, and/or to inject a fluid. The delivery channel 5 further has a fluid port 27 which may be used for introduction of other fluids. The control unit 4 is in operable connection with the proximal controlling line actuator 2 and further has a connection interface 13 for a guidance system (not shown). The connection interface 13 may be a USB port or another data connection known in the art (e.g. WiFi or Bluetooth) and may in particular be connected to a computer forming the guidance interface. A portion 101' of controlling line 101 is arranged outside of the controlling line actuator 2 and pushed therein via a hemostatic valve 12. The medical device is formed by a magnetic head 102 attached to a controlling line 101. The introducer 11 may be placed manually or with a robotic system. All the components of the delivery system 1 may be connected together when the introducer 11 is placed in the vasculature V. The delivery system 1 may then deliver the magnetic head 102 of the medical device 100 to the distal end of the introducer 11 from where the medical device enters the blood stream in the vasculature V and continues navigation autonomously. Preferably, the magnetic head 102 of the medical device 100 is directly delivered into the blood flow. The delivery system 1 may interact with the controlling line 101 such as to control its motion, e.g. the velocity of the controlling line 101, via controlling line actuator 2. Release of the controlling line 101 may let the medical device navigate, e.g. by actuation with an external magnet (not shown) or driven by blood flow, and in particular at a predetermined velocity limited by the velocity of release of the controlling line 101. Accordingly, if the release of the controlling line 101 is stopped, the motion of the medical device 100 is stopped. Rewinding the controlling line 101, e.g. by actuation of the controlling line actuator 2, may be used to draw back the medical device 100. The delivery system 1 may be used to activate other functions of the medical device 100, e.g. via the controlling line 101. For example, electrical or pneumatic power may be delivered to the medical device 100 or values measured with sensors (not shown) of the medical device 100 may be read. Electronic or mechanical components of the medical device 100 may be controlled and/or a protection (see Fig. 1c) may be removed.

Fig. 4 shows a delivery system 1 having a fluid reservoir 7 with a fluid pump 6 which delivery fluid via transmission line 19 to the housing (see Fig. 5). The control unit 4 is connected to and operates the controlling line actuator (see Fig. 5). The flow rate provided by the pump 6 may be adjusted during delivery/navigation/retraction of the medical device and may generally be tuned according to an inner diameter of the delivery system and a release velocity. A vascular flow rate may also be taken into account. For example, when using a catheter with an inner diameter between 1 and 2 mm, during the delivery, the flow rate of the fluid pump may be between 80 mL/min and 150 ml/min. During navigation, the flow rate of the delivery pump may be between 0 mL/min and 70 mL/min. During the retraction, the flow rate of the delivery pump may be between 0 mL/min and 70 mL/min.

Fig. 5 shows a detailed view of panel A shown in Fig. 4. The proximal controlling line actuator 2 is formed by spool arranged within a housing 14. The spool 15 is rotatably arranged on a shaft 17 which extends outside of the housing 14. Fluid line 19 leads into housing 14 and delivery fluid from the pump/reservoir assembly (see Fig. 4). The shaft 17 extends into housing via a rotary seal 18 which allows rotation while fluid-tight sealing of the housing 14 is maintained. The shaft 17 is formed by a motor shaft portion coupled to a corrosion resistant shaft portion. The latter passes through the rotary seal 18. The shaft may be made of stainless steel (e.g. 316L stainless steel). Due to its weight, the assembly is supported by a stand 9 which allows adjustment of the height and position of the system. The stand may be made of aluminum or titanium. The housing 14 is in fluid connection with delivery channel 5, and downstream of the delivery channel with the introducer 11 via connector 10. The rotation of the spool 15 is driven by a motor 16, but it would be conceivable to adapt the spool 15 for manual rotation as well. The motor 16 connected to the spool rotates such as to release or respool the controlling line (not shown) which enters the delivery channel 5 from housing 1. The rotation velocity of the motor 16 controls the release velocity of the medical device. An operator may input a target speed and position, which is then translated into motor movements to unspool or respool the controlling line, via a guidance system or a user interface of control unit. The medical device may be released in the vascular network with a velocity between 0.1 and 100 cm/s, preferably between 1 and 10 cm/s. The controlling line may be released by a length between 2 mm and 150 mm, preferably between 10 mm and 50 mm, at a time. In one configuration, the housing and the components inside are sterilized together and packaged.

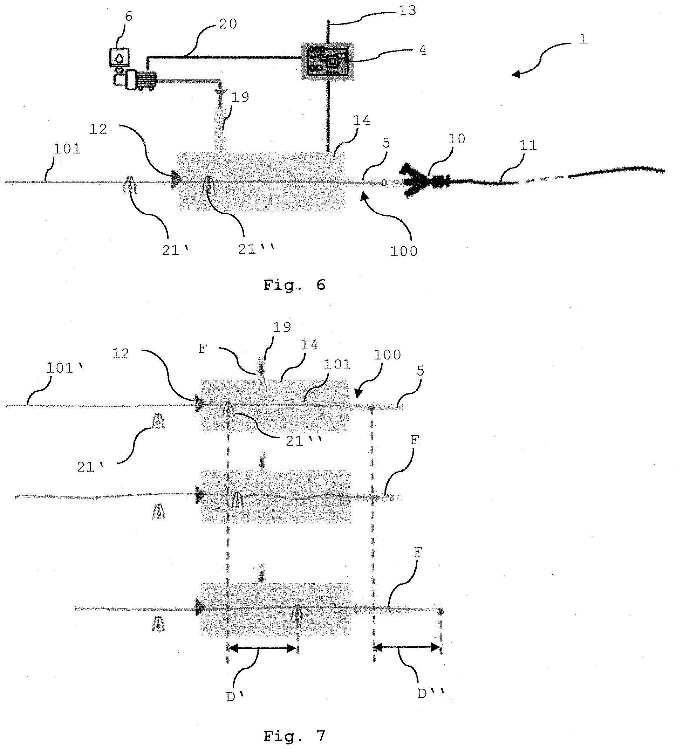

Fig. 6 shows a delivery system with clamps 21', 21'' forming a proximal controlling line actuator. Here, a control unit 4 with a interface 13 for a guidance system are present. The control unit 4, via cable 20, also controls the fluid pump 6. The fluid pump 6 delivers a saline solution to housing 14 via fluid port 19. The delivery system 1 further has a delivery channel 5 connecting the housing to introducer 11 via connector 10. A hemostatic valve 12 enables the introduction or extraction of a medical device into the housing 14. The controlling line 101 is moved with clamps 21', 21" inside and outside of the housing 14. Here, the controlling line 101 is configured as a flexible line along its entire length. A first clamp 21" is in the housing and may thus be configured as an internal motion clamp. A second clamp 21' is outside of the housing and configured as an external motion clamp. Both clamps are controls by the control unit 4, which may also control other mechatronic elements such as motors and sensors of the delivery system. Therefore, the first clamp 21" may in particular be used to pull controlling line into the housing 14, while the second clamp 21' in particular be used to pull the controlling line 101 out of the housing, via valve 12. Clamps 21', 21'' therefore enable the movement of a flexible controlling line 101 in and out of the housing 14 without requiring a pushing force. The clamps 21', 21" may exert a force, when clamping, on the controlling line 101 with a pressure between 0.05 N and 10 N and move linearly at a velocity between 0.1 cm/s and 40 cm/s, preferably between 1 cm/s and 10 cm/s. When the controlling line 101 is grabbed by the motion clamp 21', 21", a displacement of the motion clamp 21', 21" may move the controlling line 101 on a determined length with an accuracy between 0.05 mm and 4 mm, preferably around 0.3 mm. When the controlling line is not held by clamps 21', 21'', a displacement of the clamps 21', 21'' does not move the controlling line 101.

Fig. 7 shows schematically the release the controlling line 101 using the delivery system of Fig. 6. It will be understood that features already described with respect to Fig. 6 are not described repeatedly, for clarity. To release the controlling line 101, the first clamp 21" is actuated to hold the line 101 inside the housing 14. The second clamp 21' releases the controlling line 101, by unclamping outside portion 101' of line 101. As shown in the middle panel, a fluid flow F may be introduced into the delivery channel by fluid introduced by port 19. Subsequently, as shown in bottom panel, the first clamp 21" is moved distally within the housing 14 and pulls the controlling line 101 along over distance D'. As a result, the medical device 100 moves by distance D" which corresponds to distance D'. The fluid flow F through the housing 14 and into delivery channel 5 which carries the magnetic head 102 as well as the controlling line such as to reduce slack in the line due to the motion of the clamp 21". The delivery system control unit (not shown) may control the motion of the internal motion clamp 21" (speed and distance) and will therefore control the release speed and distance of the controlling line 101. The delivery system control unit may also control the flow rate delivered by the pump during that forward motion phase to ensure that the controlling line is properly carried by the flow. The position of port 19, through which fluid enters the housing 14 from the pump or reservoir, may be optimized to avoid the creation of turbulent flow and/or the accumulation of air bubbles.

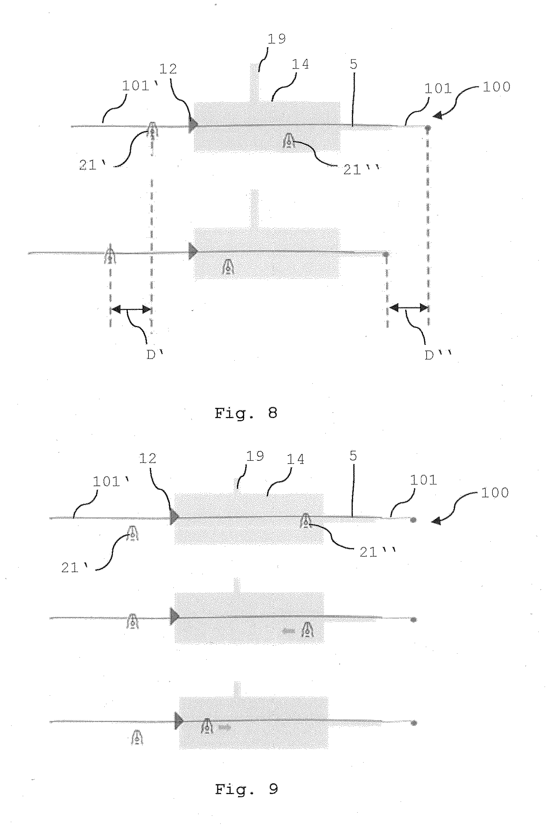

Fig. 8 shows schematically the retraction of the controlling line 101, i.e. pulling back of the controlling line 101 from an artery, using the delivery system of Figs. 6 and 7. For clarity, identical features will not be described repeatedly. For retraction, the external motion clamp 21' grabs the external controlling line portion 101' while the internal motion clamp 21" is released. The external motion clamp 21' is then moved backwards and away from the hemostatic valve 12, therefore pulling the controlling line 101 proximally.

Fig. 9 shows schematically a re-arming process of the clamps 21', 21" in a delivery system as shown in Figs. 6-8. In general, motion clamps 21', 21'' have a limited range of motion. Typically, a range between 40 mm and 400 mm, preferably between 50 mm and 100 mm, is achieved. In some applications, the clamps 21', 21" may reach the end of their motion range when moving forward or backward even though further movement of the controlling line 101 may be desired.

Here, the internal motion clamp 21' has reached the end of the housing and is not able to move further in the direction of delivery channel 5 (top panel). Therefore, the control unit (not shown) operates external motion clamp 21' to grab the external controlling line portion 101', so as to hold the controlling line 101 in place, and to release the internal motion clamp 21" to detach the clamp 21" from the controlling line 101, as seen in the middle panel. Additionally or alternatively, the hemostatic valve 12 may be adapted to selectively hold the controlling line 101. The internal motion clamp 21" may then be operated to move proximally and to grab the controlling line 101 again, as shown in the bottom panel. The internal motion clamp 21" may now move distally and release the controlling line 101 in a new cycle, e.g. as shown in Fig. 7.

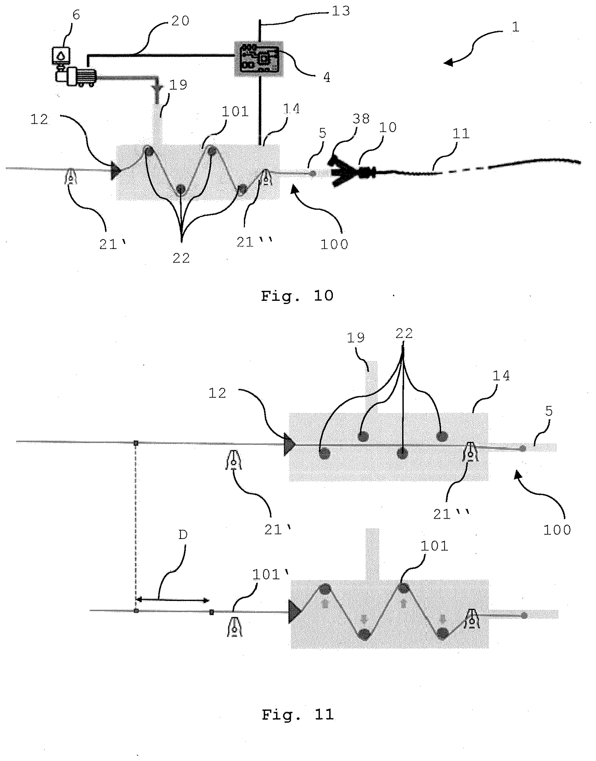

Fig. 10 shows a delivery system 1 which is substantially similar to the delivery system of Fig. 6. Identical features will not be described repeatedly, for clarity. Here, the delivery system further comprises four motion rods 22 arranged inside the housing 14. The connector has an inlet 38 for introduction of e.g. a microcatheter, a guidewire, or for injection of another fluid. Here, the motion of the controlling line 101 may be controlled by the displacement of the motion rods 22 in the housing 14. To release a portion of the controlling line 101 by a given length, the motion rods 22 load the controlling line 101 in the housing by moving away from a central axis. The Motion Rods are arranged to move linearly along a motion axis. The motion axis may be perpendicular to an axis defined by the hemostatic valve 12 and the delivery channel 5. The motion rods 22 may be adapted to move at a velocity between 1 cm/s and 30 cm/s. The motion of the motion rods 22 may be synchronized to create different motion patterns, e.g. an aligned motion or a zigzag motion. The motion rods 22 may have different cross-sectional shapes, e.g. spherical or hexagonal, and will generally have an elongated shape. The rods 22 may be coated with materials which reduce the friction with the controlling line. For example, the rods 22 may be coated with PTFE.

Fig. 11 shows schematically the operation of the delivery system 1 of Fig. 10. It will be understood that only the housing 14 is shown for clarity. When the motion rods 22 are in a disengaged state, as shown in the top panel, the controlling line 101 is substantially aligned along the central axis. Motion rods 22 are arranged on either side of the controlling line 101, here is a substantially aligned manner. It will be understood that the disengaged state may have the motion rods 22 be closer the housing 14 walls. Here, the external motion clamp 21' is released while the internal motion clamp 21" holds the controlling line 101. When the motion rods 22 are moved towards a wall of the housing on the respective other side of the controlling line 101, the controlling line 101 is formed into a zigzag pattern in the housing 14 and therefore create a portion of controlling line 101, with length D, is pulled into the housing 14 via valve 12. Therefore, controlling line 101 can be "loaded" by pulling the controlling line 101 in the housing or "unloaded" by releasing it by motion of the motion rods 22. Here, the control unit may be operated to block the controlling line 101 with the internal clamp 21" while the external clamp 21' is released, before pulling the controlling line 101, into the housing 14 via valve 12, by moving the motion rods 22 as described above.

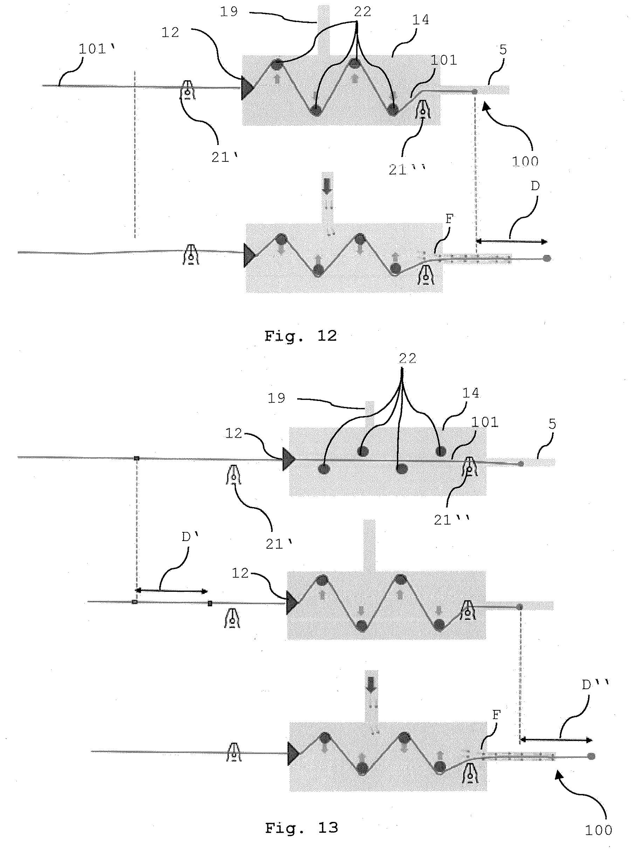

Fig. 12 shows the release of the controlling line 101 in the delivery device of Fig. 10. Here the control unit (not shown) has already performed a proximal loading of the controlling line in the housing 14, as shown in Fig. 11. To unload the controlling line 101, the control unit may block the controlling line 101 with the external clamp 21' while unblock the controlling line 101 with the internal clamp 21". Subsequently, the motion rods 22 can be moved toward the central axis, thereby releasing the controlling line which may be pulled by the fluid flow F in the delivery channel 5, thus moving the medical device 100 by distance D until the controlling line 101 is tensioned again.

Fig. 13 shows a sequence of proximal loading (as shown in Fig. 11) followed by a distal unloading (as shown in Fig. 12), resulting in a controlled movement of the controlling line 101 in the delivery channel. The control unit (not shown) may compute a desired displacement (distance, speed) of the motion rods from the desired motion of the controlling line. The desired distance may be between 1 mm and 500 mm and the desired velocity between 0.5 cm/s and 20 cm/s. The top panel shows the configuration for pulling in of the controlling line 101 essentially as shown in Fig. 11. The exterior clamp 21' is released while the interior clamp 21' holds the controlling line 101. The middle panel shows the pulling in of the controlling line 101 through valve 12, by motion of the motion rods 22, resulting in movement of the length D' by the proximal end of the controlling line 101. Finally, as shown in the bottom panel, the external clamp 21' is operated to hold the controlling line 101 while the motion rods 22 are operated to release the controlling line 101 preferably by assistance of flow F, resulting in a motion of the medical device 100 by distance D". Distance D" can be a large as length D', but it will be understood that limited motion of the motion rods 22 may also result in a shorter travelled distance of the medical device 100. The delivery system controller may control the flow rate delivered by the pump during that forward motion phase to ensure that the controlling line is properly carried by the flow. Preferably, the flow rate is between 20 mL/min and 300 mL/min.

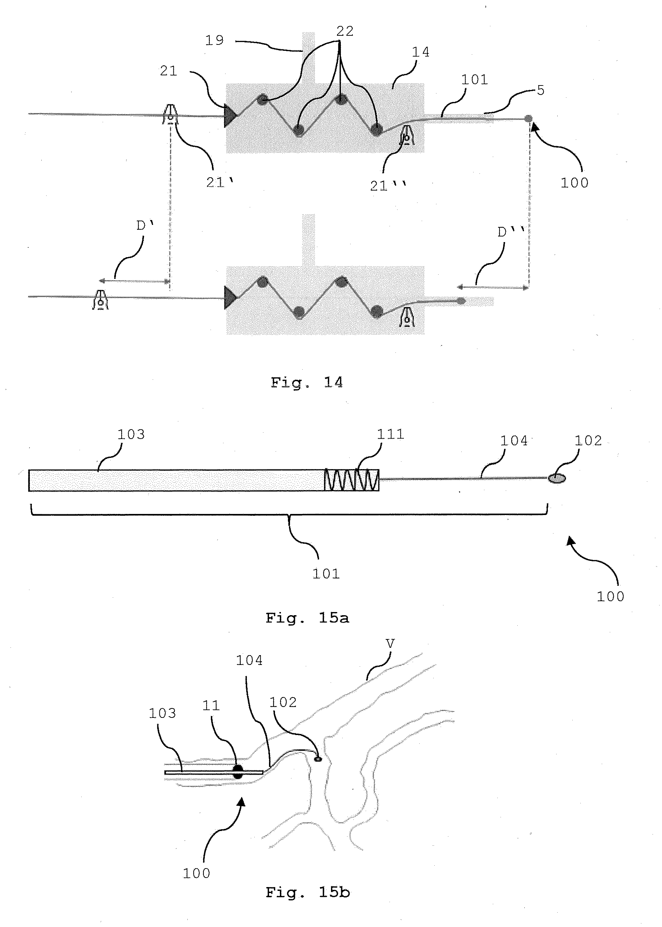

Fig. 14 shows a first method to retract the controlling line 101. Here, the controlling line 101 is pulled by means of the external clamp 21". To this end, the external clamp 21' will grab the controlling line 101 and the internal clamp 21" will release the controlling line, after which the external clamp 21' will move away from the valve 12. By moving away, the external clamp 21' will retract the controlling line 101. This motion may be done independently of the position of the motion rods 22 and typically, the motion rods 22 may stay stationary while pulling the controlling line 101 back. However, it is also possible to additionally control the pull-back rate, e.g. by accelerating it or slowing it down, by action of the motion rods 22. During the retrieving, the rods 22 may not be in contact with the control-ling line 101 to limit the friction such as to reduce the force applied by the clamps 21'.

Additionally or alternatively, the control unit may be operated hold the controlling line 101 with the external clamp 21' and to unblock the controlling line 101 with the internal clamp 21", after which the motion rods may be moved so as to pull the controlling line into the housing. Subsequently, the control unit may then block the controlling line 101 with the internal clamp 21" and grab the controlling line 101 with the external clamp 21'. The motion rods 22 may then release the controlling line 101 while the external motion clamp 21'will be moved away from the hemostatic valve 12 and pulling the controlling line 101 back.

The control unit may compute the displacement of the motion rods 22 from the desired motion of the controlling line. The motion rods 22 may be moved by a distance of 3 mm to 70 mm and with a velocity of between 1 cm/s and 50 cm/s.

It will be understood that the different methods and concepts of pulling and releasing the controlling line, as shown in Figs. 11-14, may be combined in any order and repeated as necessary.

Fig. 15a shows a medical device 100 having a magnetic head 102 and a controlling line 101 formed by a proximal rigid portion 103 and a distal flexible portion 104. Here, an element 111 with an intermediate rigidity is arranged between the distal portion 104 and the proximal portion 103. The rigidity of portion 111 may be between the rigidity of the rigid structure 103 and flexible portion 104. The portion 111 may have a bending stiffness between 500 mN·mm² and 1400 mN·mm², whereas the bending stiffness of the distal, flexible portion 104 may be between 1 mN·mm² and 100 mN·mm². The rigidity may be tuned by increasing or decreasing the thickness of the controlling line 101, e.g. by progressively increasing the diameter of the controlling line from portion 104 to portion 103.. The rigid portion 103 may, optionally, be formed as a hollow tube. The hollow tube has an outer diameter between 0.3 mm to 4 mm, preferably between 0.9 mm and 2.5 mm. The inner diameter is between 0.2 mm and 2 mm, preferably between 0.5 mm and 1.2 mm. The tube can be made of metals such as nickel-titanium alloys or polymers or a PTFE, Tygon, PEBAX (commercial name, a combination between polyamide and polyether) or combination. The tube can be made of several tubes setup together. As an example, the outer tube is made of PEBAX. The inner tube is made of PTFE. In one configuration, the distal part of the hollow "rigid structure" is more flexible. Such performance can be achieved by reducing the thickness of the tube, removing one inner tube, or by adding a flexible element made of an elastomer (Silicon, Polyurethane).

Once in the vascular network, the µ-robot will be under tension due to the blood flow.

Fig. 15b shows the medical device 100 of Fig. 15a being placed in a vasculature V. The introducer 11 is moved to a location in the vasculature V where the medical device 100 is released. The displacement of the rigid structure 103 induces a slack of the distal portion 104 of the controlling line 101. This slack is transferred into a movement of the magnetic head 102 which may be carried by a blood flow pushed at least by the flow.

To help the steering of the proximal portion 103 along the path followed by the magnetic head 102, the distal portion 104 of medical device, preferably the magnetic head 102 may, rotate on itself under external magnetic field variation. The rotation induces a winding of the distal portion 104 of the controlling line 101 which in turn pulls on the distal part of the proximal part 103. To allow the winding, the proximal portion 103 may be moved forward during the rotation.

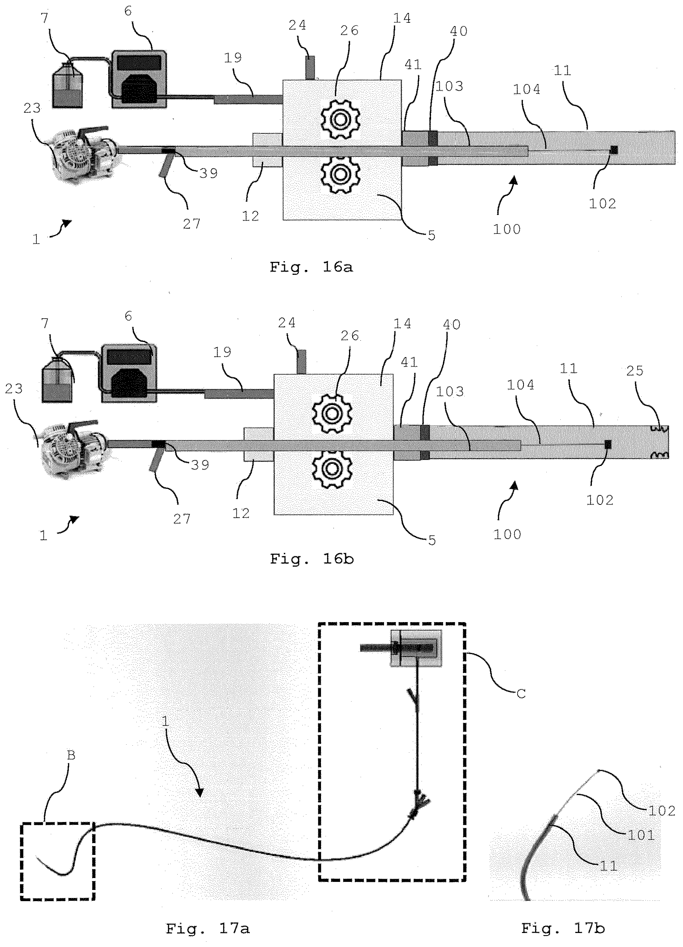

Fig. 16a a shows a delivery system 1 with a medical device 100 similar to the one shown in Fig. 15a. Here, the housing 14 has a purge valve 24 and receives a fluid from reservoir 7 delivered to port 19 by pump 6, as shown e.g. in Figs. 4 and 5. Similarly, a hemostatic valve 12, as shown in previous embodiments, is arranged on housing 14 and will not be described in more detail here. Here, the delivery channel 5 is substantially formed by the housing 14. Gear wheels 26 are arranged within the housing 14 and are adapted to interact with the proximal portion 103 of controlling line 101, by pushing distally or pulling proximally. A vacuum pump 23 is connected to the proximal portion 103 and adapted for providing a suction action therein, which may be transmitted through distal portion 104 and magnetic head 102 (see Fig. 1b). A selecting valve 39 is used to connect the vacuum pump 23 to the proximal portion 103 of the tethered medical device 100. The selecting valve 39 is used to pass a guidewire, a solution or both in the hollow pushable structure. In this embodiment, the introducer 11 is connecting through a connector 40 and a delivery channel 41 to the housing.

Fig. 16b shows a delivery system 1 which is substantially similar to the embodiment of Fig. 16a. Here, the introducer 11 has a magnetic tip formed by an electromagnet 25 at a distal end. The electromagnet 25 may be used to bend the distal part of the introducer 11 with a magnetic actuator (not shown) for its setting in the vascular network. Alternatively, the magnetic tip of the introducer 11 may be a permanent magnet. Here, the introducer 11 further comprises an electromagnet 25 arranged at a distal end of the catheter portion 8.

Fig. 17a shows an overview of a delivery system 1.

Fig. 17b shows a detailed view of panel B of Fig. 17a. Here, the introducer 11 is visible after the magnetic head 102 and controlling line 101 have exited a distal end of the introducer 11.

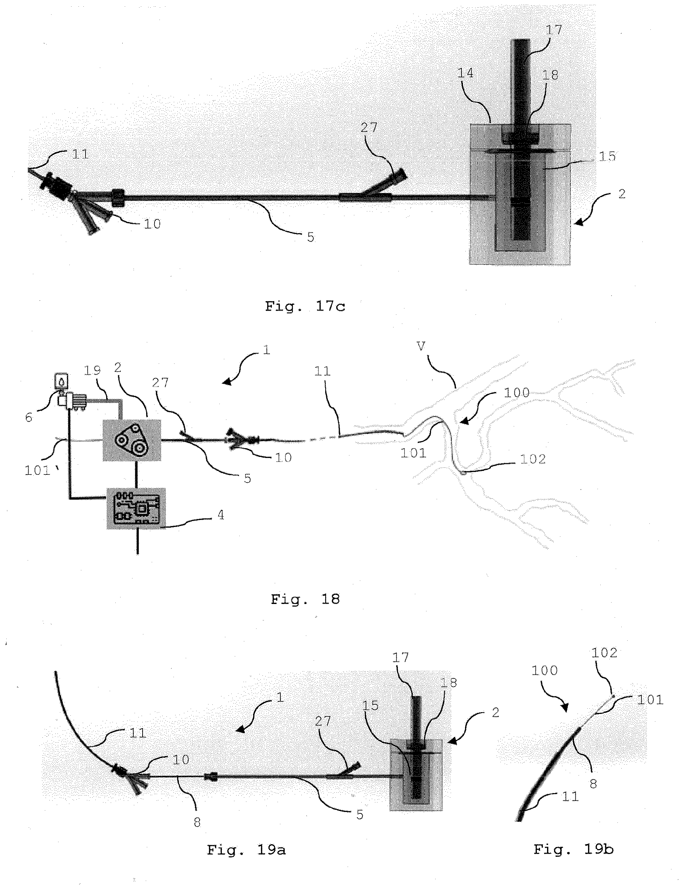

Fig. 17c shows a detailed view of panel C of Fig. 17a. Introducer 11, delivery channel 5 with port 27 substantially correspond to other embodiments shown above. Here, housing 14 is connected to the delivery channel 5 and houses a spool 15 with shaft 17 which is connected to spool 15 via rotary seal 18 and thus forming the controlling line actuator 2.

As the medical device 100 is mainly moved by the flow in the vascular network, a flow may be used to move it along the introducer 11. To move the medical device 100 inside the introducer 11, the controlling line 101 is moved forward by means of actuator 2 and a flow is applied. To create the flow, e.g. a saline-based solution may be used. The flow may be applied after the displacement of the controlling line 101 or at the same time. It is particularly advantageous to apply the flow at the same time as the movement of the controlling line 101 such as to avoid the formation of slack in the introducer 11. The flow can be induced manually, e.g. with a syringe, or by a pump.

Fig. 18 shows an example of a delivery system which is similar to the embodiment of Fig. 17a. Here, the delivery system 1 integrates a peristatic pump 6 which is controlled via the control unit 4. The controlling line actuator 2 is formed by in a similar fashion as e.g. shown in Fig. 14 and an external portion 101' of the controlling line 100 is moved through the actuator 2. The pump 6 is connected to the controlling line actuator 2 via channel 19. The flow induced by the pump 6 can be continuous or pulsatile and the flow rate is regulated according to the controlling line actuator velocity. The flow rate is tuned to ensure a progressive displacement of the µ-robot at a continuous velocity, preferably between 1 cm/s and 15 cm/s. The control unit 4 is connected to the pump 6 and operates the pump according to these principles.

Fig. 19a shows a delivery system 1 having a controlling line actuator 2 and a delivery channel 5 substantially corresponding to the embodiment of Figs. 17a-17c and will not be described repeatedly. Here, the delivery channel 5 is connected to microcatheter 8 and which forms an extension of the delivery channel 5. The microcatheter 8 is slidably inserted into connector 10 which leads into introducer 11. For delivery, a distal part of the medical device may be releasably attached to a distal extremity of the microcatheter 8. The lumen of the catheter 8 has a diameter of 1 mm but may be anywhere in the range of 400 µm and 1800 µm, preferably between 900 um and 1100 µm. The microcatheter 8 is moved inside the introducer 11 up to the vascular network. The microcatheter 8 may be pushed by an operator or by a robotic system.

Fig. 19b shows a distal portion of the delivery system 1 in more detail. Here, the introducer 11 forms an opening through which catheter 8 extends. The medical device 100 exits the catheter 8. In particular, the microcatheter may be perfused with flow to reduce the friction of the controlling line 101 during the navigation. At a target site, the magnetic head 102 may be pushed away from the microcatheter 8, e.g. by flushing the microcatheter 8 with a saline solution and/or by pushing with a guidewire (not shown). The magnetic head 102 may be moved at least by 1 mm, preferably by 5 to 10 mm.

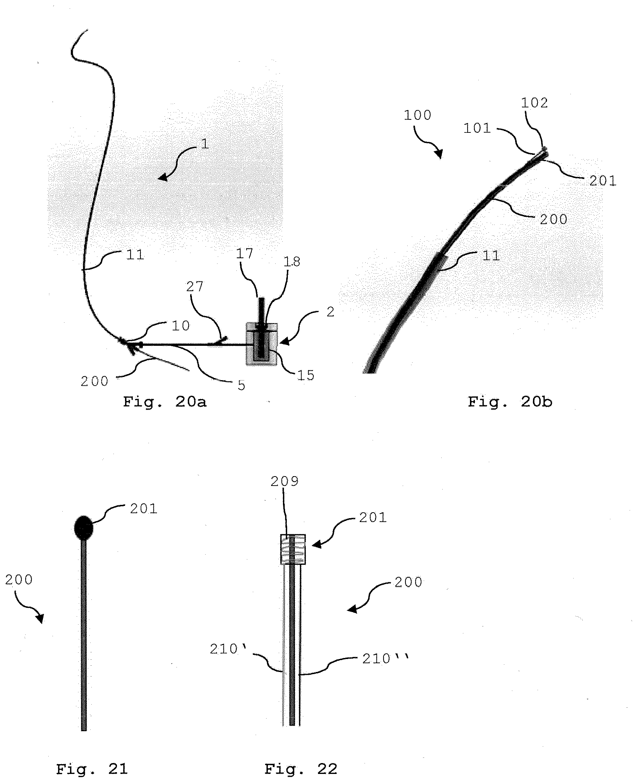

Fig. 20a shows the delivery system 1 of Fig. 17a, wherein a guidewire 200 is inserted through a second opening of the connector 10 and extends through the introducer 11.

Fig. 20b shows a detailed view of a distal-most part of the delivery system 1. The medical device 100, in the form of magnetic head 102 and controlling line 101, extend out of the introducer 11. Furthermore, guidewire 200 also extends out of introducer 200. Guidewire 200 has a magnetic element 201 which selectively attaches, via magnetic forces, to magnetic head 102 and allows pushing or pulling of the magnetic head 102 via guidewire 200. The magnetic head 201 has a length of 15 mm and is made of a permanent magnet. The magnetic guidewire 200 is pushed manually by an operator (not shown).

Fig. 21 shows an example of a magnetic guidewire 200 made of a thin wire and having a magnetic element 201 formed as a permanent magnet.

Fig. 22 shows an alternative embodiment of a magnetic guidewire 200 having an magnetic element 201 configured as an electromagnet. Here, the electromagnet is formed by a coil 209 which is connected to a power source by first cable 210' and second cable 210". Applying a voltage via cables 210', 210" turns on the electromagnet 209.

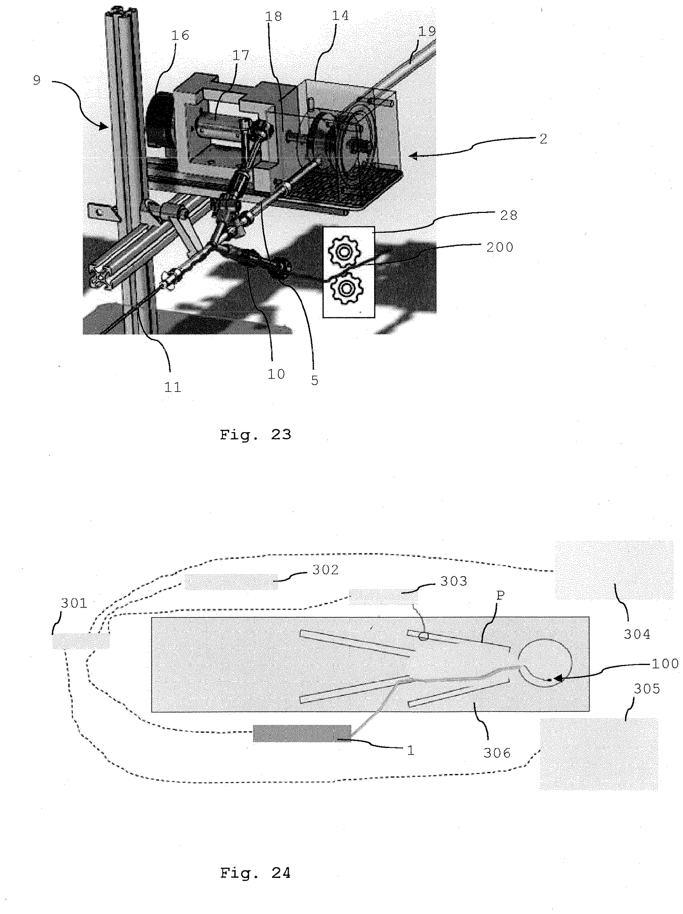

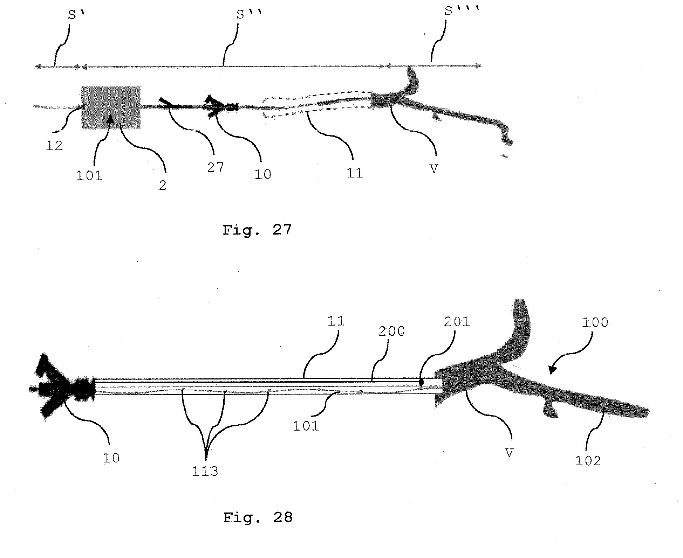

Fig. 23 shows a portion of a delivery device 1 in the region of housing 14 and connector 10. The shown embodiment substantially corresponds to the embodiment of Fig. 5 and identical features will not be described repeatedly for clarity. Here, additionally, a guidewire 200 as shown in Fig. 22 is inserted via connector 10. Here, the magnetic guidewire 200 is moved and activated by a robotic system comprising gear wheels 28 for actuating the guidewire and controlled by control unit 4. This robotic system allows forward and backward movement of the magnetic guidewire 200 and, separately, to turn on and off the magnetic field of an electromagnet(see Fig. 22). When a magnetic head of the guidewire 101 is magnetically attached to a magnetic portion, e.g. the head of the medical device 102 , the magnetic guidewire 200 with the actuator 28 may move the distal part of the medical device (see Fig. 28).

Fig. 24 shows treatment system for guiding a medical device 100 which was delivered by a delivery system 1 according to the invention. A patient P is placed on a table 306. The medical device 100 is steered by magnetic actuator 305. An imaging system 304 provides images of the medical device 100 inside the patient and may include e.g. ultrasound imaging. A patient sensor 303 is provided to e.g. measure a heart rate and blood pressure of the patient P. A tracking system 302 allows to monitor motion of the patient P, the magnetic actuator 305 and the imaging system 304. A controller 301 is operably connected to tracking system 303, imaging system 304, magnetic actuator 305, delivery system 1 and patient sensor 303. The medical device 100 moves with the different forces such as blood flow, actuators, and magnetic forces from the magnetic actuator 305. The magnetic actuator 305 here is made of a permanent magnet. Additionally or alternatively, electro-magnetic coils or electro-permanent coils may be used. The magnetic actuator 305 may be arranged on a robotic arm (not shown). The displacement velocity of the robotic arm may be slower than the actuation of the delivery system 1 to ensure that the medical device 100, in particular the magnetic head, is positioned within the magnetic field of magnetic actuator 305. In one configuration, to limit the friction of medical device inside the introducer of delivery system, a flow may be injected into the introducer with a moderate flow rate, for example between 5 mL/min and 50 mL/min.

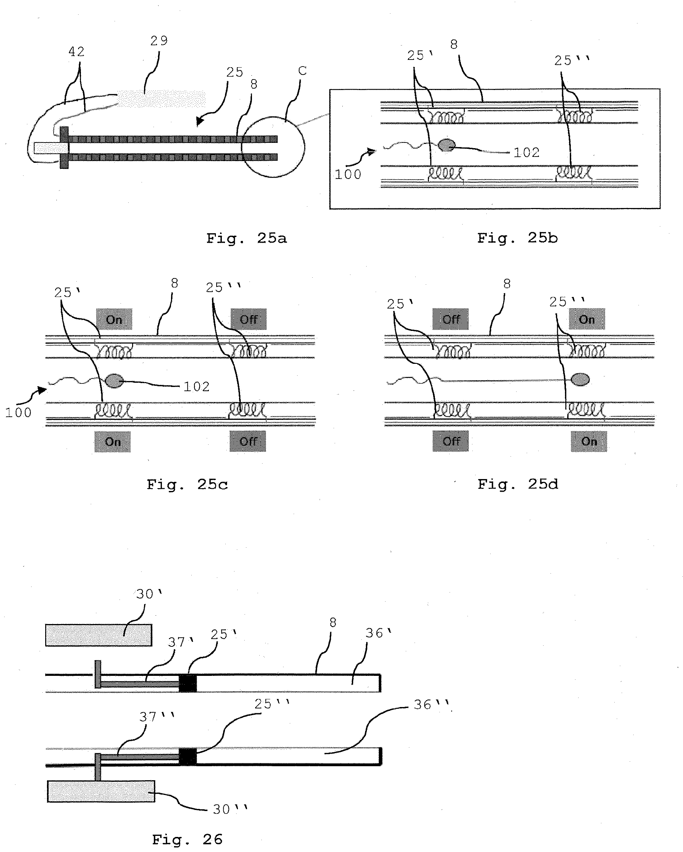

Fig. 25a shows schematically a catheter 8, for use in a delivery device, e.g. as shown in Figs. 19a-19b. The catheter 8 has power source 29 with an integrated controller which is connected to catheter 8 via electric cables 42. The catheter 8 comprises a plurality of internal magnetic elements 25 which allow the displacement of the medical device (not shown) inside a catheter lumen, as will be shown in Fig. 25b.

As shown in Fig. 25b, pairs of electromagnetic coils 25', 25'' are arranged along the catheter axis at a distance from 5 to 40 mm. For clarity, it will be understood that only a first coil pair 25' and a second coil pair 25'' is shown, but further coil pairs may be arranged along the catheter axis. Similarly, single coils may be used instead of pairs or more than two coils may be used at one position along the catheter axis. The outer diameter of the catheter is between 2 mm and 5 mm, preferably between 3 mm and 4 mm. The lumen diameter is between 1.5 mm and 2.7 mm. The activation of the coils 25', 25" may be synchronized with the displacement of the medical device 100, e.g. by turning on the respective coil which is passed by the medical device 100 next (see Figs. 25c-25b). In another embodiment, the coil 25' may be activated in a repulsive mode once the magnetic head has passed that coil. Other combinations of coil magnetic field management could be used to optimize the movement of the magnetic head.

Fig. 25c shows the catheter 8 of Fig. 25a-25b with a medical device 100 passing therethrough. First coil pair 25' is turned on and attracts magnetic head 102 thereto. When the magnetic head reaches first coil 25', the second coil pair 25'' is turned on and the first coil pair 25' is turned off. As a result, as shown in Fig. 25d, the magnetic head 102 is moved toward the second coil pair 102.

Fig. 26 shows an alternative embodiment of a magnetic catheter 8 having a slidable magnetic elements 25', 25' which are movable by means of actuators 30', 30''. The magnetic elements 25', 25'' are attached to wires 37', 37'' which are fixed to a spool each (not shown), each of which is part of actuators 30', 30". The spool may be rotated manually or by a motor. The wires 37', 37" are placed in lumen 36', 36'' to move back and forth. As a result, the magnetic elements 25', 25'' may be placed at the distal part of the catheter 8, partially into the lumen of the blood vessel and may also assist in retrieving the medical device into the catheter 8 because the magnetic element may pull the magnetic head of the medical device back into the catheter 8. Thus, the risk of blockage of the medical device during the retrieving may be reduced.

Alternatively, the sliding by actuators 30', 30' may be achieved by any other means known in the art. For example, an inflation system may be used to push the magnetic elements 25', 25'' forward and backward. A motorized slider may be arranged in the catheter lumen.

The magnetic elements 25', 25'' may be a permanent magnets or an electro magnets. In the case of a permanent magnet, to release the medical device (not shown) into the blood flow, a drag force higher than the magnetic force may be induced by the pump by increasing the flow rate (see e.g. Fig. 7). Preferably, the magnetic elements 25', 25" are formed as electro-magnets. Once the current is removed, the medical device may be pushed only by a moderate drag force into the blood flow.

Fig. 27 shows, with respect to delivery system 1 which substantially corresponds to the embodiment of Fig. 3 (control unit not shown here), schematically different segments of a controlling line 101 to which reference will be made in the following. An external segment S' of controlling line 101 is arranged outside the first actuator 2 and pulled therein. A delivery system segment S" may denominate a section of the controlling line arranged between the controlling line actuator up until the introducer 11. The second actuator may contribute to the pulling of the medical device in that section S". A blood flow segment S‴ may refer to the portion of the controlling line arranged in the blood flow.

The blood flow segment S‴ of the medical device may be moved at least with one force, or several forces, which may include the drag force, the magnetic force and/or a combination thereof.

The delivery system segment S" may be subjected to friction forces in the delivery system which may exceed a pulling force exerted on the blood flow segment S‴. The friction force may include dry and dynamic friction, adhesion forces and electrostatic forces. As will be shown below, some embodiments provide a reduction of friction forces in the system. Some of the features may reduce friction forces on the blood segment S‴ as well.

The external segment S' may be pulled by the controlling line actuator 2. A valve 12 may between the external segment S' and the delivery system segment S". Hence, because of the valve 12, an increased force may be needed to pull the controlling line 101 from the external segment S".

Fig. 28 shows a delivery system, here only shown partially with connector 10 and introducer 11 while a medical device 100 is navigating a patient's vasculature V. Here, the controlling line 101 is configured with a plurality of magnetic elements 113. A guidewire 200 having an electromagnetic element 201 is introduced, via connector 10, into introducer 11 and has rigidity which is sufficient to be pushable. The magnetic elements 113 may have different shapes, e.g. spherical, ovoid, cylindrical, square, and/or paralepidid. Spheres may have a diameter between 100 µm and 800 um. The magnetic elements 113 may be comprise or consist of hard ferromagnetic materials such as Nd-Fe-B, soft ferromagnetic materials such as iron alloys, ferrimagnetic materials such as Fe₃O₄ compounds, and/or a combination thereof. Magnetic elements 113 may be encapsulated in a polymer matrix, e.g. in polyurethane. The magnetic elements can be attached to the controlling line, for example, by gluing, knots, or welding, or embedded in the controlling line (e.g. during manufacturing of the controlling line).

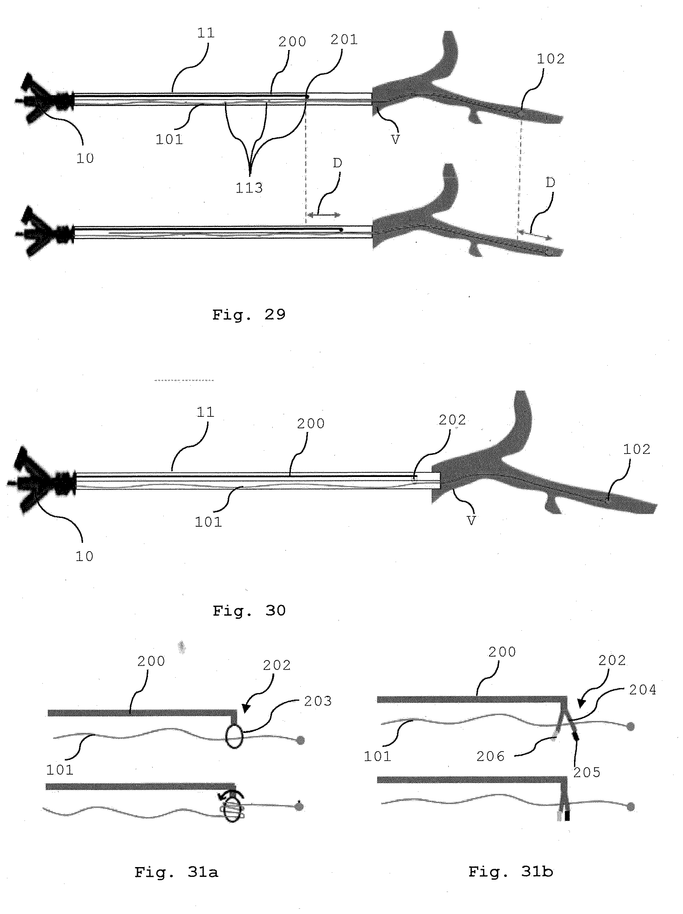

Fig. 29 shows schematically the advancement of controlling line 101 in the system of Fig. 28. When a magnetic element 113 is in the vicinity of magnetic element 201 of guidewire, the electro-magnet 201 may be turned on the resulting attraction force may be used to pull the controlling line 101 forward by pushing the guidewire 200 by a distance D, which ultimately results in motion by magnetic head 102 by same distance D. It will be understood that the time until the magnetic head 102 reaches its new position, moved by distance D, depends on the strength of the drag force exerted by the blood flow, but is typically in the range of a few seconds. Subsequently, the magnetic element 201 may be turned off, the guidewire 200 pulled back and the process repeated with another magnetic element 113.

Fig. 30 shows an embodiment of a delivery system which is similar to the delivery system of Fig. 29. Here, the guidewire 200 has a mechanical attachment element.202, instead of magnetic element, and may grab and release the controlling line 101. To pull the controlling line 101 in the delivery system 1, the guidewire 200 may be moved to position the mechanical attachment element 202 at the desired location along the controlling line in the introducer 11 and subsequently grab the controlling line 202. The control unit of the delivery system may release the controlling line 101 and, simultaneously, push forward the guidewire 200 to pull the controlling line 200, while the controlling line 101 and the magnetic head portion 102 may also be moved by a blood flow or other fluid flow. Once the mechanical attachment element 202 has reached the extremity of the delivery system, the mechanical attachment element 202 may release the controlling line 101, the guidewire 200 is pulled back to a more proximal position of the controlling line 101 and a new pulling cycle may be starting, as described above.

Figs. 31a-31d show schematically different embodiments of a mechanical attachment elements 202 which may, e.g., be used on a guidewire as shown in Fig. 30. The mechanical attachment element 202 may be activated by different energies such as mechanical energy, electricity, pneumatic energy, and/or active materials (e.g. nitinol). The control of the positioning of the mechanical attachment element 202 may be done by fluoroscopy, a localization sensor, and/or motion models.

The mechanical attachment element 202-may have a solution to keep the controlling line in the area of action of the mechanical attachment element 202, for example a circle in which the controlling line can be guided and move freely.

The controlling line may have features facilitating the grabbing such as a handle or a protuberance. In general, mechanical attachment is advantageous because such additional features, while advantageous in some circumstances, are not necessary and any controlling line may be grabbed by mechanical attachment element 202.

Fig. 31a shows a mechanical attachment element 202 on a guidewire 200 in the form of a twisting ring 203 through which the controlling line 101 may extend. By rotation of ring 203, the controlling line 101 may be pulled back.

Fig. 31b shows an electro-magnetic clamp 204 which has a tweezer 204 which may be compressed by attraction of Ferromagnetic element 205 and electromagnet 206 to grab the controlling line 101.

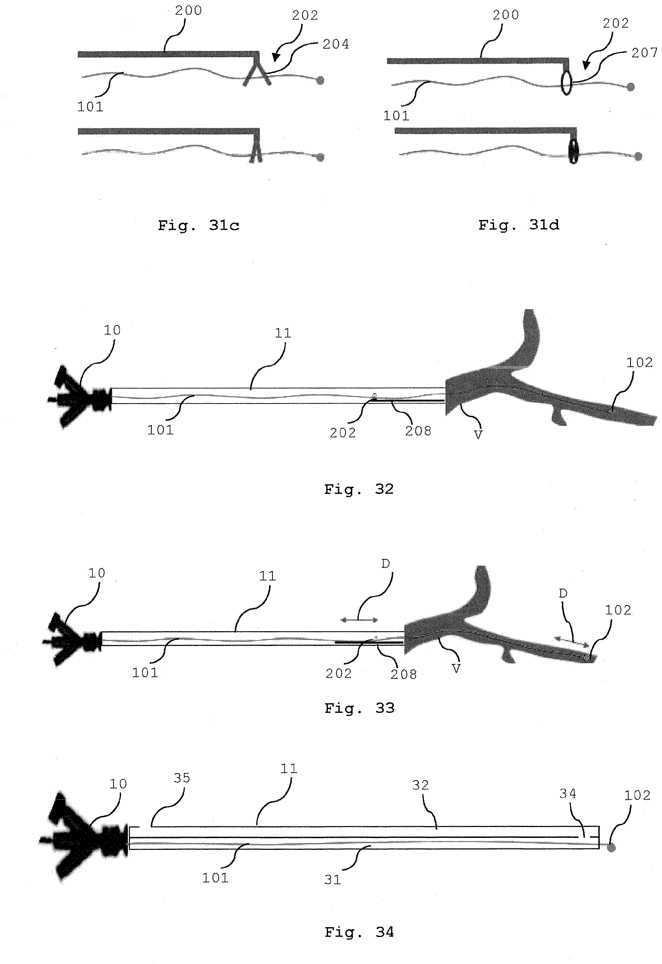

Fig. 31c shows a mechanical attachment element 202 in the form of a mechanical clamp 204.

Fig. 31d shows a mechanical attachment element 202 having a balloon 207 which may grab the controlling line 101 by inflation.

Fig. 32 shows a delivery system similar to the embodiment of Fig. 30. Here, instead of a guidewire, a motion actuator 208 with a mechanical attachment element 202 arranged thereon is placed at a distal end of the introducer 11. The mechanical attachment element 202 may be any mechanical attachment element 202 as shown in Figs. 31a-31d. The motion actuator 208 comprises a motion mechanism to move backward or forward the mechanical attachment element 202 at the extremity of the introducer 11.

The control unit of the delivery system may control the activation of the mechanical attachment element 202 and motion mechanism.

Fig. 33 shows the delivery system of Fig. 32, wherein the motion mechanism of mechanical attachment element 202 moved, by distance D, in a distal direction while controlling line 101 was held by mechanical attachment element 202. As a result, the controlling line 101 was moved forward by distance D as well, resulting in a forward travel of magnetic head 102 by distance D as well.

Pulling the proximal section of the controlling line 101 may be used to help release the distal section of the controlling line 101. To pull the controlling line in the delivery system, the mechanical attachment element 202 is disengaged to release the controlling line 101 and the motion mechanism moves the mechanical attachment element 202 away from the distal extremity of the delivery system. The mechanical attachment element 202 is activated to grab the controlling line 101. The delivery system control unit may then release the controlling line 101 and activate the motion mechanism so as to move the mechanical attachment element 202 toward the distal extremity of the delivery system, while the distal section of the controlling line and the medical device may be moved with the flow. Once the motion mechanism reaches the extremity of the motion range, the delivery system controller may stop the release of the controlling line 101 and the motion of the motion mechanism. Iterating this cycle may allow to pull a long section of the controlling line 101 despite the limited range of motion of the motion mechanism. The mechanical attachment element 202 may be moved by a mechanical rod inserted in the delivery system, a pneumatic solution to push or pull the mechanical attachment element 202, and/or ropes (e.g. using pulleys) to move forward or backward the grabbing element.

Fig. 34 shows a delivery system having a dual lumen formed by first lumen 31 and second lumen 32 connected by opening 34 at a distal end of the introducer 11. The outer diameter of the introducer 11 may between 2 mm and 5 mm, preferably between 2.5 mm and 4.5 mm. The first lumen 31, which is for the navigation of the medical device 100, may have a diameter between 1 mm and 2.5 mm. The second lumen 32 for the flow circulation may have a diameter between 0.5 mm and 1.5 mm. Once a flow is present in the first lumen 31 in a distal direction and, via opening 34 back in proximal direction in second lumen 32, the liquid may be passed by a filter (not shown) in order to avoid or reduce the entry of blood components into the delivery system. Due to the liquid circulation, the amount of liquid injected by the delivery system into the patient may be reduced.

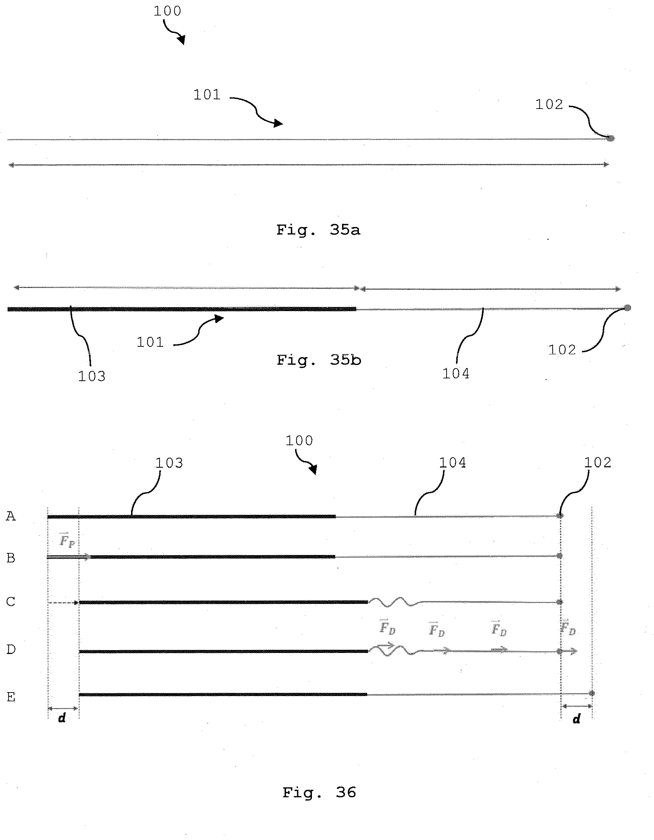

Fig. 35a shows a medical device 100 having controlling line 101 consisting of one section. A magnetic head 102 is attached to distal end of the controlling line 101. The controlling line 101 is flexible and adapted to not allow movement of the magnetic head 102 by pushing the controlling line 101.

Fig. 35b shows a medical device 100 having a controlling line 101 with a proximal section 103 and a distal section 104. The proximal section 103 is pushable,i.e. its rigidity is higher than that of the distal section 104 and allows to move, by pushing on a proximal end, the proximal section 103 of the controlling line 101. The distal section 104 is flexible and may in particular correspond to the controlling line 101 shown in Fig. 35a. The magnetic head 102 is attached to a distal end of the distal section 104.

Fig. 36 shows schematically a forward movement of a medical device 100 as shown in Fig. 35b by pushing on the proximal section 103 of the controlling line 101. Panel A shows the device 100 at an initial position. As shown in panel B, a force F_{P} is applied to the proximal end of the proximal section 103, which leads to a displacement of the proximal section 103 of the controlling line as shown in panel C. The distal section 104 therefore has some slack. Subsequently (panel D), the flow force F_{D} pushes the magnetic head 102 and the distal section 104 forward, leading to a fully displaced device as shown in panel E. Moving of a device as shown here may require two actuation mechanisms, wherein one mechanism pulls a the distal section 104 of the controlling line 101 and/or the magnetic head 102 and another mechanism is used to push the proximal section 103 of the controlling line. The actuation of the distal section 104 may be done by blood flow, but another actuation mechanism may be necessary if not blood flow is present or exerts an insufficient force. The movement induced by both actuation mechanism may be synchronized.

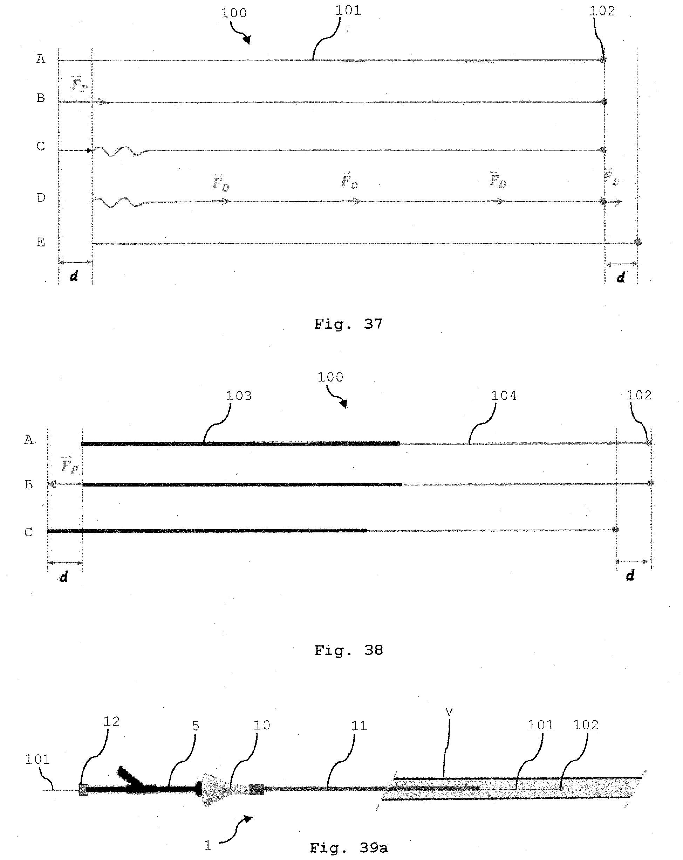

Fig. 37 shows schematically a forward movement of a medical device 100 as shown in Fig. 35a by pushing on the controlling line 101. Panel A shows the initial position and panel B a pushing force F_{P} being exerted, leading to a displacement by distance d. The pushing introduces slack substantially at the proximal end of the controlling line 101, as shown in panel C, which is then pulled forward by the blood flow force F_{D} (panel D), leading to the final displaced position (panel E).

Fig. 38 shows a pulling back of the medical device 100 of Fig. 35. Panel A shows an initial position. A pulling force FP is exerted on the proximal end of the proximal section 103 (panel B), leading to a backwards movement by distance d of the magnetic head 102 (panel C) .

Fig. 39a shows a delivery system 1 which is substantially similar to the delivery system shown in Fig. 3. The controlling line 101 is introduced through the hemostatic valve 12 and exits the delivery system 1, at a distal end of the intruder 11, into a patient's vessel V

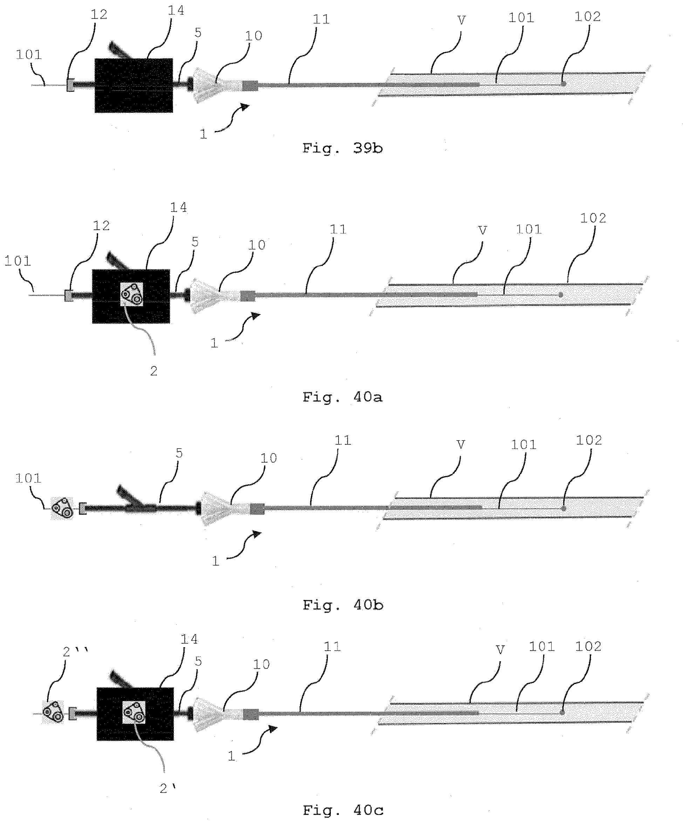

Fig. 39b shows a delivery system 100 which is substantially similar to the embodiment shown in Fig. 39a. Here, the delivery channel 5 is partially a housing 14 which provides a larger enclosure.

Figs. 40a-40c show different embodiments of delivery systems 1 which are similar to the embodiments of Figs. 39a and 39b. The embodiments of Figs. 40a-40c differ from one another in the arrangement of the controlling line actuator 2.

Fig. 40a shows an embodiment of a delivery system 1 wherein the controlling line actuator 2 is arranged within the housing 14.

Fig. 40b shows an embodiment of a delivery system 1 without a housing but having a controlling line actuator 2 arranged outside of the delivery channel 5, at a proximal position relative to the hemostatic valve 12.

Fig. 40c shows an embodiment of a delivery system 1 wherein the controlling line actuator consists of a first portion 2' arranged within the housing and a second portion 2" arranged outside the delivery channel 5 at a position proximal of the hemostatic valve 12.

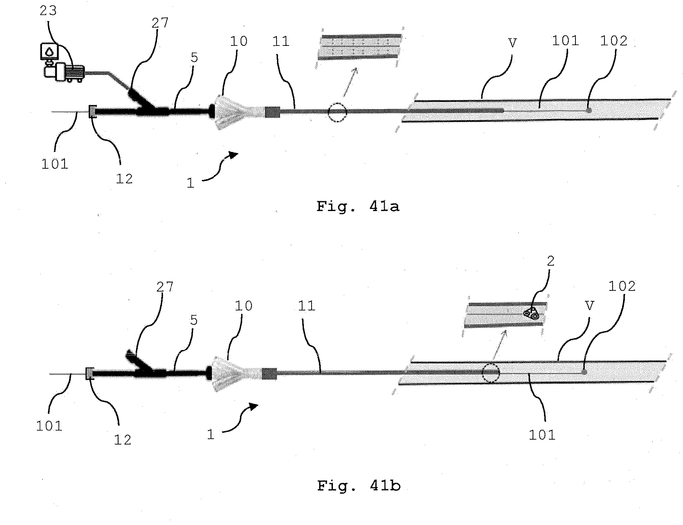

Figs. 41a-b show two embodiments of a delivery system 1 which are similar to the delivery systems shown in Figs. 40a-40c. Identical features with the same reference numerals will not be described again, for the sake of clarity, and the skilled person will understood that the shown embodiments here differ in the actuation of the controlling line 101. The skilled person will understand, however, that the actuation modes of Figs. 40a-40c may be combined with the actuation of Figs. 41a-41b, e.g. either one of the shown embodiments here may in addition also have further controlling line actuator as shown in e.g. any of Figs. 40a-40c.

Fig. 41a shows a delivery system 1 which comprises a fluid pump 23 which is in fluid connection, via port 27, with the delivery channel 5. Introduction of a fluid introduces a flow inside the introducer 11 which in turn actuates the controlling line 101 at a distal end and thus moves the controlling line 101 forward. The fluid pump 23 therefore acts as a distal controlling line actuator.

Fig. 41b shows a delivery system 1 having a controlling line actuator 3, for example as shown in Figs. 30-33, arranged at a distal end of the introducer 11. The controlling line actuator 3 is thus adapted to pull the controlling line 101 from a distal end of the delivery system 1, i.e. acting as a distal actuator..

## Claims

1. A delivery system (1) for a medical device (100),
wherein said medical device (100) is adapted for floating in or moving with a fluid in a patient's body (P) and has a controlling line (101) and a distal magnetic head (102), the controlling line (101) having a proximal portion (103) and a distal portion (104) and wherein at least the distal portion (104) is flexible and attached to the magnetic head (102),
the delivery system (1) comprising at least a first actuator (2), a second actuator (3), a control unit (4), and a delivery channel (5) with a longitudinal axis (L),
wherein the delivery channel (5) is adapted for at least partially receiving the medical device (100) therein,
wherein the first actuator (2) is adapted for moving the proximal portion (103) of the controlling line (101) in at least two directions along the longitudinal axis (L), and
wherein the second actuator (3) is adapted for exerting a force, along a distal direction of the longitudinal axis (L), on the distal magnetic head (102) and/or the distal portion (104) of the controlling line (101), and wherein
the control unit (4) is adapted to control the first actuator (2) depending on the second actuator (3).

2. The delivery system of claim 1, further comprising a fluid pump (6) and/or a fluid reservoir (7), preferably wherein the control unit (4) is adapted for injecting and/or withdrawing fluid from the delivery channel (5), via a fluid inlet (19), preferably wherein a positive or negative pressure is provided, particularly preferably a pressure with a predefined value.

3. Delivery system according to claim 2, wherein the second actuator (3) is formed by the fluid pump (6) and/or a fluid reservoir (7).

4. Delivery system according to any one of the preceding claims, wherein the second actuator (3) comprises or consists of a guidewire (200), wherein the guidewire (200) has, preferably at a distal end, at least one of a magnetic element (201), preferably an electromagnetic element (209), a spool (15), pliers (204, 205), a clamp (202), and a balloon-based mechanism (207).

5. Delivery system according to any one of the preceding claims, further comprising an introducer (11) which is fluid connection with the delivery channel (5), wherein the second actuator (3) comprises or consists of at least one magnetic element (25', 25") and/or a mechanical attachment element (202) arranged in the introducer (11), preferably at a distal region of the introducer (8).

6. A delivery system for a medical device (100), preferably a delivery system (1) according to any one of the preceding claims,
wherein said medical device (100) is adapted for floating in a fluid in a patient's body (P) and has a controlling line (101) and a distal magnetic head (102), the controlling line (101) having a proximal portion (103) and a distal portion (104) and wherein at least the distal portion (104) is flexible and attached to the magnetic head (102),
the delivery system (1) comprising a first actuator (2), a delivery channel (5), and a control unit (4), wherein
the first actuator (2) is adapted for releasing and/or drawing back the controlling line (101), and wherein the first actuator (2) is at least partially arranged within the delivery channel (5 and/or a housing (14), preferably wherein the housing (14) forms part of the delivery channel (5), and/or the delivery channel (5) is attached to the housing (14) such as to form an inner channel which is in fluid communication with an inner volume of the housing (14), and wherein
the control unit (4) is adapted to control the first actuator (2) such as to release and/or draw back the controlling line (101), preferably via the inner channel.

7. Delivery system (1) according to any one of the preceding claims, wherein at least one of the delivery channel and the housing is in fluid communication with a purge valve.

8. Delivery system (1) according to one of claims 6 or 7, further comprising a fluid pump (6) and/or a fluid reservoir (7), preferably wherein the control unit (4) is adapted for injecting and/or withdrawing fluid from the delivery channel (5) and/or the housing, via a fluid inlet (19) , preferably wherein a positive or negative pressure is provided, particularly preferably a pressure with a predefined value.

9. Delivery system according to any one of the preceding claims, wherein the first actuator (2) comprises or consists of at least a first holder (21"), preferably wherein the first holder (21") is arranged external of the delivery channel (5).

10. Delivery system (1) according to any one of the preceding claims, wherein the first actuator (2) comprises or consists of at least a second holder (21'), preferably wherein the second holder (21') is arranged inside the housing and/or the delivery channel (5).

11. Delivery system (1) according to any one of claims 9 or 10, wherein the control unit (4) is adapted for controlling the operation of the first and/or second holder (21', 21"), preferably dependent on the movement of the controlling line (101).

12. Delivery system (1) according to any one of the preceding claims, comprising an introducer (11), preferably an introducer (11) formed by a catheter (8), wherein the introducer (11) is connected or connectable to the delivery channel (5) by a connector (10), preferably a connector (10) having an inlet (38).

13. Delivery system (1) according to any one of the preceding claims, wherein the first actuator (2) comprises or consists of a spool (15).

14. Delivery system (1) according to any one of the preceding claims, further comprising a motor (16) for operating the first actuator (2), preferably wherein the motor (16) is arranged outside the delivery channel (5) and/or the housing and/or is in operable connection with the first actuator (2) via a contactless gear and/or rotary seal (18).

15. Delivery system (1) according to any one of the preceding claims, wherein the delivery channel (5) has a hemostatic valve (12) adapted for receiving the controlling line (101) and allowing sliding of the controlling line (101) in a sealed manner.

16. Delivery system (1) according to any one of the preceding claims, wherein the first actuator (2) further comprises at least one rod (22) arranged within the delivery channel (5) and adapted for tensioning and/or releasing the controlling line (101).

17. Delivery system (1) according to any one of the preceding claims, further comprising a medical device (100) which is adapted for floating in a fluid in a patient's body (P) and has a controlling line (101) and a distal magnetic head (102), the controlling line (101) having a proximal portion (103) and a distal portion (104) and wherein at least the distal portion (104) is flexible and attached to the magnetic head (102), and wherein the proximal portion (103) of the controlling line (101) is in operable connection with the first actuator (2).

18. Delivery system (1) according to claim 17 and any one of claims 1 to 5, wherein the controlling line (101), preferably the distal portion (104), is in operable connection with the second actuator (3).

19. Delivery system according to any one of the preceding claims, wherein at least a portion of an inner surface of the delivery channel (5) and/or the introducer is coated with an anti-friction coating.

20. Delivery system according to any one of the preceding claims, wherein the control unit is adapted for operating the first actuator (2) and/or the fluid pump (6) in dependence of a magnetic force acting on the medical device (100) by a magnet (305) adapted for steering the medical device (100) in a patient's body (P).

21. Guidewire (200) for arrangement or introduction in the catheter portion (11, 8) of a delivery device (1) according to any one of the preceding claims, wherein the guidewire (200) has a first end adapted for interacting with the medical device (100) such as to pull or push the medical device (100) through the catheter portion (11, 8), preferably wherein the first end comprises one of a magnet (209) and a clamp (204).

22. Catheter device (8) for delivering a medical device (100) into a patient's body (P),
said medical device (100) having a magnetic head portion (102) and a controlling line (101), said medical device (100) being adapted for floating in a fluid in a patient's body (P).

23. The catheter device (8) according to claim 22, comprising a plurality of 'selectively activatable magnetic elements (25', 25") arranged along a longitudinal axis of the catheter device (8).

24. The catheter device (8) according to any one of claims 22 or 23, further comprising a holder (202, 208) adapted to hold a controlling line (101), preferably wherein said holder (202, 208) is arranged at a distal end of the catheter device (8).

25. Delivery system (1) according to any one of claims 1 to 20, wherein the delivery channel (5), is connected to a catheter device (8), preferably a catheter device (8) according to any one of claims 23 or 25, wherein an inner channel of the catheter device (8) forms, at least partially, an extension of the delivery channel (8).

26. A medical device (100) for introduction into a patient's body (P), comprising a magnetic head portion (102) and a controlling line (101), wherein a plurality of magnetic elements (113) are arranged on or in the controlling line (101) along a longitudinal axis, preferably wherein the magnetic elements (113) do not substantially affect the mechanical properties of the controlling line (101).

27. Delivery system (1) according to any one of claims 1-20 or 25, comprising a medical device (100) according to claim 26.

28. A method of treating a patient (P), comprising the steps of introducing a medical device (100) in a patient's body (P), wherein the medical device (100) is adapted for floating in or moving with a fluid in a patient's body (P) and has a controlling line (101) and a distal magnetic head (102), preferably a medical device (100) according to claim 26, the controlling line (101) having a proximal portion (103) and a distal portion (104) and wherein at least the distal portion (104) is flexible and attached to the magnetic head (102), preferably wherein using a delivery system (1) according to any one of claims 1 to 20 or 25 or 27, wherein a motion of the medical device (100) is synchronized with the heart cycle, preferably wherein an advancement of the medical device is started during a systole phase.
